# EUROPEAN PATENT APPLICATION

(11) **EP 3 889 602 A1**
(43) Date of publication of application: **06.10.2021**
(21) Application number: 20166918.1
(22) Date of filing: 30.03.2020
(51) Int. Cl.: G01N 33/50, G01N 33/574

(54) **MR1 LIGANDS AND PHARMACEUTICAL COMPOSITIONS FOR IMMUNOMODULATION**

(71) Applicant: Universität Basel, 4001 Basel (CH)
(72) Inventor: The designation of the inventor has not yet been filed
(74) Representative: Schulz Junghans Patentanwälte PartGmbB

(57) **Abstract**

The invention relates to a method for modulating an interaction between an MR1 polypeptide and an MR1-specific T cell receptor molecule, whereby a MR1 polypeptide is contacted with a MR1 ligand compound that is a nucleobase adduct product reflecting a state of metabolic distress of a eukaryotic cell.

The invention further relates to the use of compounds identified as MR1 ligands in vaccination or modulation of an MR1-restricted immune response.

## Description

The present invention relates to ligands specifically presented by MR1 molecules to MR1-specific T cells. These ligands are derivatives or analogues of nucleic acid forming bases, particularly ribonucleoside and deoxyribonucleoside adducts occurring in eukaryotic cells under certain conditions. The invention further relates to pharmaceutical preparations and methods for use of such ligands in treatment and research. The invention further relates to pharmaceutical preparations provided with the aim of increasing presentation of MR1 ligands in clinical situations where such increased presentation of MR1 ligands is of clinical benefit.

### Background of the Invention

MR1 (Uniprot ID 95460) is a non-polymorphic MHC class I-like protein that is expressed at low levels on the surface of most cell types. MR1 is highly conserved across multiple species, with human and mouse MR1 sharing >90% sequence homology at the protein level.

The inventors recently published their work confirming the existence of human T cells that recognize tumor-associated antigens (TAAs) presented by MR1 (Lepore et al., ELIFE 6, DOI:10.7554/eLife.24476). These novel T cells participate in tumor immune surveillance, thus representing novel tools for cancer immunotherapy. The antigens recognized by these MR1-specific T cells, however, remain unknown.

Adoptive therapy with donor- or patient-derived T cells engineered to express T-cell receptors (TCRs) specific for selected TAAs represents a promising and safe strategy to induce clinically relevant anti-tumor immune response in cancer patients. Targeting TAAs bound to MR1 non-polymorphic antigen presenting molecules might overcome this constraint and in principle be applicable to all patients bearing tumors expressing MR1. The use of tumor-reactive TCRs that recognize MR1-presented antigens might also have the advantage of complementing anti-tumor responses mediated by MHC-presented peptide antigens, excluding cross-competition of TAAs for binding to the same type of presenting molecule. In addition, this strategy may provide the possibility of targeting antigens of different nature on the same tumor cells, thus minimizing the potential occurrence of tumor escape variants under selective immune pressure.

The absence of information about the nature of the presented antigens, and the lack of tools available to probe, analyze and modulate the presentation of MR1, its interaction with antigen and with cognate TCRs, presents an obstacle in the development of improved MR1-centred immune-oncological treatments.

Therefore, the identification of MR1-presented TAAs and the identification and isolation of MR1-restricted TCRs recognizing these antigens might have important implications for cancer immunotherapy.

Based on the above-mentioned state of the art, the objective of the present invention is to provide means and methods to modulate MR1-TCR interaction to enable and improve clinical applications of MR1-based immunotherapy. This objective is attained by the subject-matter of the independent claims of the present specification.

### Summary of the Invention

To the knowledge of the inventors, the present invention for the first time identifies MR1-specific eukaryotic antigen compounds presented to MR1 T cells in the context of an MR1-restricted immune response to intracellular antigens arising from metabolic states of the eukaryotic cell, thereby facilitating these compounds' uses for therapeutic/preventive and diagnostic or research purposes.

Consequently, the invention provides nucleobase-adduct MR1 ligand compounds, in many embodiments, nucleoside adducts or analogues, for use in methods harnessing the specificity of MR1-TCR interactions (with the MR1 molecule presenting the specific MR1 ligand compound to the T cell:
i) a method to stimulate MR1 ligand compound-specific T cells, and induce a prophylactic or therapeutic immune response,
ii) a method for the identification and isolation of T cells reactive to the compounds as presented on MR1,
iii) a method to classify metabolically altered cells, including but not limited to tumor cells, according to the presence of the compounds.

The invention further relates to a method to modulate (increasing or decreasing) the quantity of MR1 ligand compounds in the cells or presented by the cells, by pharmacological intervention leading to accumulation or disposal of the MR1 ligand compounds in the cells.

In another aspect, the present invention relates a pharmaceutical composition comprising at least one of the compounds of the present invention or a pharmaceutically acceptable salt thereof and at least one pharmaceutically acceptable carrier, diluent or excipient.

### Detailed Description of the Invention

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *MR1* in the context of the present specification refers to either the MR1 gene (Entrez 3140) or the MR1 gene product (Uniprot Q95460).

The term *MR1T cell* in the context of the present specification refers to a T cell that expresses a T cell receptor capable of binding specifically to an MR1 molecule presenting an antigen molecule as specified herein.

The term *MR1T cell receptor* in the context of the present specification refers to a T cell receptor capable of binding specifically to an antigen presented, for example by a cancer cell, in association with an MR1 molecule.

The expression of a marker such as MR1 may be assayed via techniques such as fluorescence microscopy, flow cytometry, ELISPOT, ELISA or multiplex analyses.

In the present specification, the term *positive*, when used in the context of expression of a marker, refers to expression of an antigen assayed by a fluorescent labelled antibody, wherein the fluorescence is at least 5% higher (≥5%), in median fluorescence intensity in comparison to staining with an isotype-matched antibody which does not specifically bind the same target. Such expression of a marker is indicated by a superscript "plus" (⁺), following the name of the marker, *e.g.* MR1⁺.

In the present specification, the term *negative*, when used in the context of expression of a marker, refers to expression of an antigen assayed by a fluorescent labelled antibody, wherein the median fluorescence intensity is less than 5% higher than the median fluorescence intensity of an isotype-matched antibody which does not specifically bind the same target. Such expression of a marker is indicated by a superscript minus (⁻), following the name of the marker, *e.g.* MR1⁻.

In the context of the present specification, the term *checkpoint modulator agent* encompasses checkpoint inhibitory agents (particularly checkpoint inhibitory antibodies) and checkpoint agonist agents (particularly checkpoint agonist antibodies).

In the context of the present specification, the term *checkpoint inhibitory agent* or *checkpoint inhibitory antibody* is meant to encompass an agent, particularly an antibody (or antibody-like molecule) capable of disrupting the signal cascade leading to T cell inhibition after T cell activation as part of what is known in the art the immune checkpoint mechanism. Non-limiting examples of a *checkpoint inhibitory agent* or *checkpoint inhibitory antibody* include antibodies to CTLA-4 (Uniprot P16410), PD-1 (Uniprot Q15116), TMIGD2 (Uniprot Q96BF3), BTLA (Uniprot Q7Z6A9), CD160 (Uniprot 095971), Lag-3 (Uniprot P18627), TIGIT (Uniprot Q495A1), CD96 (Uniprot P40200), TIM-3 (Uniprot Q8TDQ0), CEACAM1 (Uniprot P13688), SIRP alpha (Uniprot P78324), CD200R (Uniprot Q8TD46), KIR family (including Uniprot proteins Q99706, P43628, P43626, Q8NHK3, P43627, Q8N109, B0L652, Q86U48, B0L653, A0A191URI1, Q6H2H3), ILT family , or to PD-L1 (Uniprot Q9NZQ7), PD-L2 (Uniprot Q9BQ51), VISTA (Uniprot Q9H7M9), B7H3 (CD276; Uniprot Q5ZPR3), CD80 (Uniprot P33681), CD86 (Uniprot P42081), B7-H4 (Uniprot P42081), TNFRSF14 (HVEM, CD270, Uniprot Q92956), CD155 (Uniprot P15151), Galectin9 (Uniprot 000182), CD200 (Uniprot Q8TD46).

In the context of the present specification, the term *checkpoint agonist agent* or *checkpoint agonist antibody* is meant to encompass an agent, particularly but not limited to an antibody (or antibody-like molecule) capable of engaging the signal cascade leading to T cell activation as part of what is known in the art the immune checkpoint mechanism. Non-limiting examples of receptors known to become upregulated upon T cell activation include CD25 (Uniprot P01589), CD122 (Uniprot P14784) and CD137 (4-1BB; Uniprot Q07011). The term *checkpoint agonist agent* or *checkpoint agonist antibody* encompasses agonist antibodies to CD28 (P10747), ICOS (Q9Y6W8), CD137 (4-1BB; Uniprot Q07011), Light (HVEM, Uniprot O43557), OX40 (P23510), GITR (Q9Y5U5), DNAM-1 (CD226, Uniprot Q15762), 2B4 (CD244, Uniprot Q9BZW8), DR3 (Q93038), NKp80 (KLRF1, Uniprot Q9NZS2).

The term *C₁-C₄ alkyl* in the context of the present specification relates to a saturated linear or branched hydrocarbon having 1, 2, 3 or 4 carbon atoms, wherein in certain embodiments one carbon-carbon bond may be unsaturated and one CH₂ moiety may be exchanged for oxygen (ether bridge) or nitrogen (NH, or NR with R being methyl, ethyl, or propyl; amino bridge). Non-limiting examples for a C₁-C₄ alkyl are methyl, ethyl, propyl, prop-2-enyl, n-butyl, 2-methylpropyl, *tert*-butyl, but-3-enyl, prop-2-inyl and but-3-inyl. In certain embodiments, a C₁-C₄ alkyl is a methyl, ethyl, propyl or butyl moiety.

The term *unsubstituted Cₙ alkyl* when used herein in the narrowest sense relates to the moiety -CₙH₂ₙ- if used as a bridge between moieties of the molecule, or -CₙH₂ₙ₊₁ if used in the context of a terminal moiety.

The terms *unsubstituted Cₙ alkyl* and *substituted Cₙ alkyl* include a linear alkyl comprising or being linked to a cyclical structure, for example a cyclopropane, cyclobutane, cyclopentane or cyclohexane moiety, unsubstituted or substituted depending on the annotation or the context of mention, having linear alkyl substitutions. The total number of carbon and -where appropriate- N, O or other hetero atom in the linear chain or cyclical structure adds up to n.

The term *substituted alkyl* in its broadest sense refers to an alkyl as defined above in the broadest sense, which is covalently linked to an atom that is not carbon or hydrogen, particularly to an atom selected from N, O, F, B, Si, P, S, Cl, Br and I, which itself may be -if applicable- linked to one or several other atoms of this group, or to hydrogen, or to an unsaturated or saturated hydrocarbon (alkyl or aryl in their broadest sense). In a narrower sense, *substituted alkyl* refers to an alkyl as defined above in the broadest sense that is substituted in one or several carbon atoms by groups selected from amine NH₂, alkylamine NHR, imide NH, alkylimide NR, amino(carboxyalkyl) NHCOR or NRCOR, hydroxyl OH, oxyalkyl OR, oxy(carboxyalkyl) OCOR, carbonyl O and its ketal or acetal (OR)₂, nitril CN, isonitril NC, cyanate CNO, isocyanate NCO, thiocyanate CNS, isothiocyanate NCS, fluoride F, choride Cl, bromide Br, iodide I, phosphonate PO₃H₂, PO₃R₂, phosphate OPO₃H₂ and OPO₃R₂, sulfhydryl SH, suflalkyl SR, sulfoxide SOR, sulfonyl SO₂R, sulfanylamide SO₂NHR, sulfate SO₃H and sulfate ester SO₃R, wherein the R substituent as used in the current paragraph, different from other uses assigned to R in the body of the specification, is itself an unsubstituted or substituted C₁ to C₁₂ alkyl in its broadest sense, and in a narrower sense, R is methyl, ethyl or propyl unless otherwise specified.

The term *amino substituted alkyl or hydroxyl* substituted alkyl refers to an alkyl according to the above definition that is modified by one or several amine or hydroxyl groups NH₂, NHR, NR₂ or OH, wherein the R substituent as used in the current paragraph, different from other uses assigned to R in the body of the specification, is itself an unsubstituted or substituted C₁ to C₁₂ alkyl in its broadest sense, and in a narrower sense, R is methyl, ethyl or propyl unless otherwise specified. An alkyl having more than one carbon may comprise more than one amine or hydroxyl. Unless otherwise specified, the term "substituted alkyl" refers to alkyl in which each C is only substituted by at most one amine or hydroxyl group, in addition to bonds to the alkyl chain, terminal methyl, or hydrogen.

The term *carboxyl substituted alkyl* refers to an alkyl according to the above definition that is modified by one or several carboxyl groups COOH, or derivatives thereof, particularly carboxylamides CONH₂, CONHR and CONR₂, or carboxylic esters COOR, with R having the meaning as laid out in the preceding paragraph and different from other meanings assigned to R in the body of this specification.

The term *halogen-substituted alkyl* refers to an alkyl according to the above definition that is modified by one or several halogen atoms selected (independently) from F, Cl, Br, I.

A *carboxylic ester* is a group -CO₂R, with R being defined further in the description. A carboxylic amide is a group -CONHR, with R being defined further in the description.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

In the present specification, the following abbreviations are used: APC, antigen-presenting cell; β2m, β2 microglobulin; DC, dendritic cell; HLA, human leukocyte antigen; HPLC, highpressure liquid chromatography; IFN-γ, interferon-γ; IL-13, interleukin 13; mAb, monoclonal antibody; MAIT cell, mucosal associated invariant T cell; MFI, median fluorescence intensity; MHC, major histocompatibility complex; MR1, MHC class I-related molecule 1; MR1T cell, MR1-restricted T cell; MS, mass-spectrometry; PBMC, peripheral blood mononuclear cell; reactive oxygen species, ROS; TAA, tumor-associated antigen; TCR, T cell receptor; TIL, tumor-infiltrating lymphocyte.

Any of the aspects of the invention relate to the use of an MR1 (major histocompatibility complex class I-related gene protein 1) ligand compound described by any one of the following general formulas 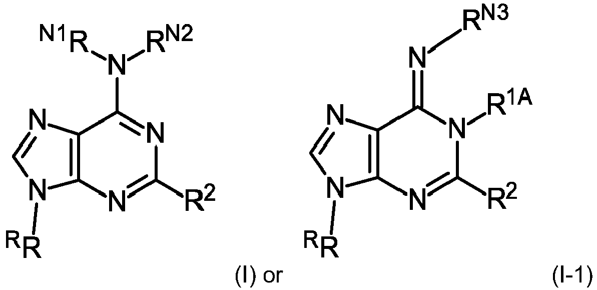 or 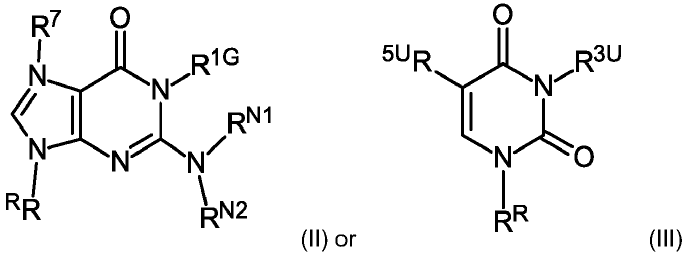 or 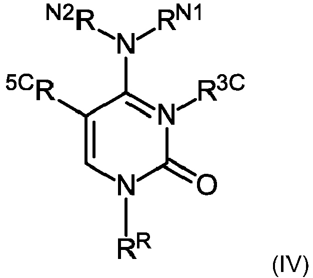 or wherein
- R^{1A} is H or methyl, R^{1G} is H or methyl;
- R² is selected from H, methyl and -S-methyl;
- R^{3U} and R^{5U} are selected from H and methyl; R^{3C} may be methyl or no atom attached to the N methyl (if R^{3C} is methyl, the N3 nitrogen formally bears a positive charge), and R^{5C} is selected from H and methyl;
- R^{N1} and R^{N2} are each independently selected from H and unsubstituted or substituted C₁ to C₈ alkyl, particularly R^{N1} and R^{N2} are both H or C₁ to C₃ alkyl, or R^{N1} is H or C₁ to C₃ alkyl (particularly R^{N1} is H or methyl) and R^{N2} is selected from methyl, threonylcarbamoyl, isopent-2-enyl, cis-hydroxyisopent-2-enyl, 3-oxo-1-propenyl (propenal), and hexa-1,3,5-triene-1,1,3-tricarbaldehyde (or R^{N1}, R^{N2} and the nitrogen together form a 2-oxa-8-azabicyclo [3.3.1] nona-3,6-diene-4,6-dicarbaldehyde bi-annular system);
   or
- R^{N3} and R^{1A} together, or R^{N2} and R^{3C} together, or R^{N1} and R^{1G} together form an unsubstituted or C₄ to C₁₆-2-oxo-alkyl-substituted or ω-carboxy-2-oxo-alkyl-substituted imidazole ring together with two atoms of the purin ring and the extracyclic amine nitrogen, or
   R^{N3} and R^{1A} together, or R^{N2} and R^{3C} together, or R^{N1} and R^{1G} together are - C(CH₃)OH-CHOH- or C(R')OH-CH₂-CHOH- with R' being selected from H, CH₃, CH(OH)C₂H₅, C₂H₅ and C₄H₉;lipid peroxidation product adducts or R^{N1} and R^{1G} together with N1 and C2 of the purin ring and the extracyclic amine nitrogen, form a unsubstituted pyrimidine, or R^{N1} and R^{1G} together with N1 and C2 of the purin ring and the extracyclic amine nitrogen, form a 12-oxo-5,6,10,12-tetrahydro-3H-6,10-methano[1,3,5]oxadiazocine ring system;
   and
- R⁷ is selected from an unbound electron pair and methyl (if R⁷ is methyl, the N7 nitrogen formally bears a positive charge);
- R^{R} is selected from H, 1'-ribosyl, 5'phospho-1ribosyl, 1'-(2'-O-ribosyl-5"-phosphate)ribosyl, 1'-(2'-O-methyl)ribosyl;

The inventors' present understanding is that the "modification of the nucleoside" represented by moieties R^{1A}, R^{2,} R^{N1}, R^{N2}, R^{N3}, etc. that constitute the structural difference between the adduct and the parent nucleoside, facilitates interactions with the MR1 pocket, thus generating a stable complex with MR1. No structural information has been obtained yet to provide a rationale of how the different adducts are inserted within the MR1 pocket. Different adducts might assume orthogonal positions. For example, while adenosine-containing adducts might have the two rings parallel to the alfa helices of MR1, the guanosine adducts might position the two rings in a perpendicular manner. This might explain while addition of residues in the two different positions of the primary amine of adenosine and guanosine are both compatible with binding. Data obtained from crystal structures of MR1 binding bacterial MR1 ligands support this notion.

The natural nucleobases and their ribosyl or deoxyribosyl derivatives, i.e. adenine, adenosine, deoxyadenosine, guanine, guanosine, deoxyguanosine, uracil, uridine, deoxyuridine, thymine, thymidine, deoxythymidine, cytosine and cytidine and deoxycytidine are not deemed to be encompassed by the scope of general formulas I, II, III and IV, and are not deemed useful for the methods and uses disclosed herein.

Also, the inventors have found that the compounds queuosine, wybutosine, hydroxywybutosine, pseudouridine, and(2R,3S,4R,5R)-2-(hydroxymethyl)-5-(6-(methylthio)-9H-purin-9-yl)tetrahydrofuran-3,4-diol can be used for the practice of the invention, as can be modifications thereof formed in the spirit of the invention as disclosed herein. 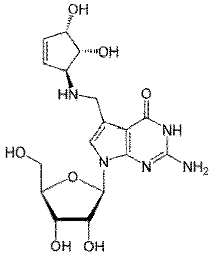 Queuosine: C₁₇H₂₃N₆O₇; MW 409.39
Toronto Research Chemicals, N925205 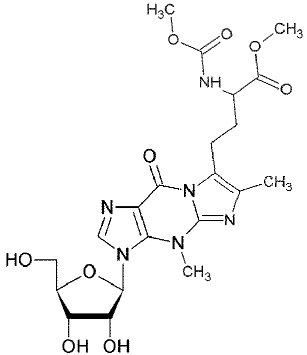 Wybutosine; C₂₁H₂₈N₆O₉; MW 508.49 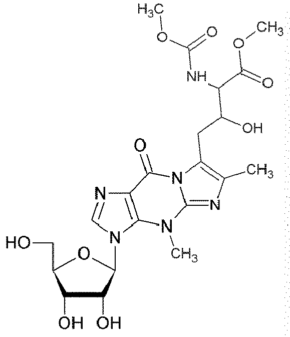 Hydroxywybutosine, C₂₁H₂₃N₆O₁₆; MW: 524.49 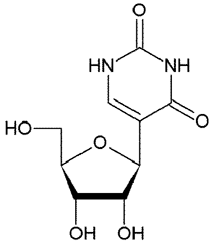 5-(3,4-dihydroxy-5-(hydroxymethyl)tetrahydrofuran-2-yl)pyrimidine-2,4(1*H*,3*H*)-dione; Pseudouridine, Psi; C₉H₁₂N₂O₆; MW: 244.20 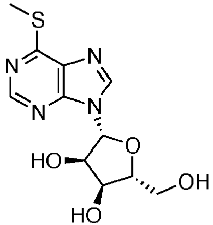 6-Methylmercaptopurine, (2*R*,3*S*,4*R*,5*R*)-2-(hydroxymethyl)-5-(6-(methylthio)-9H-purin-9-yl)tetrahydrofuran-3,4-diol; 6-MMP; C₁₁H₁₄N₄O₄S; MW: 298.32

In certain embodiments of any of the nucleoside derivative compounds disclosed herein as MR1 ligands, R^{R} is described by the general formula (V) 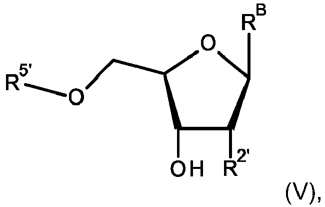 wherein R^{B} is the bond connecting the moiety to the N⁹ nitrogen of I, Ia or II, or to the N¹ nitrogen of II or IV, R^{2'} is selected from H, OH, OCH₃, O-ribosyl and O-ribosyl-5"-phosphate, and R⁵ is selected from H and PO₃²⁻.

In certain particular embodiments of any of the nucleoside derivative compounds disclosed herein as MR1 ligands, R^{R} is described by the general formula (Va) 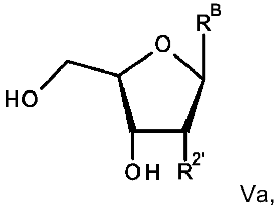 wherein R² is selected from H, OH, O-methyl, O-1-ribosyl and O-1-(5-phospho)-ribosyl. Va with R² being OH is shown as Vb; Va with R² being O-1-(5-phospho)-ribosyl is shown as Vc.

In certain more particular embodiments of any of the nucleoside derivative compounds disclosed herein as MR1 ligands, R^{R} is described by the general formula (Vb) or (Vc)

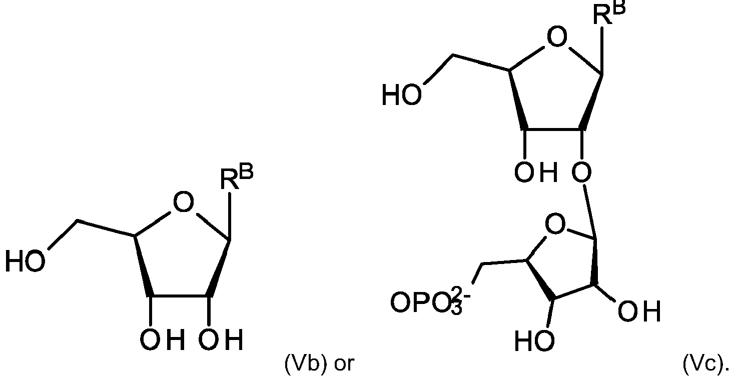

It is understood that the phosphate on the 5" ribosyl oxygen of Vc can be in acid form (OPO₃H₂) or as a hydrogen phosphate or phosphate salt together with a suitable anion.

In certain embodiments of any of the nucleoside derivative compounds disclosed herein as MR1 ligands, the MR1 ligand compound is described by any one of the following general formulas 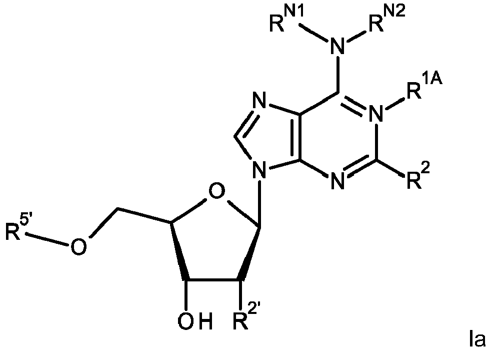 or 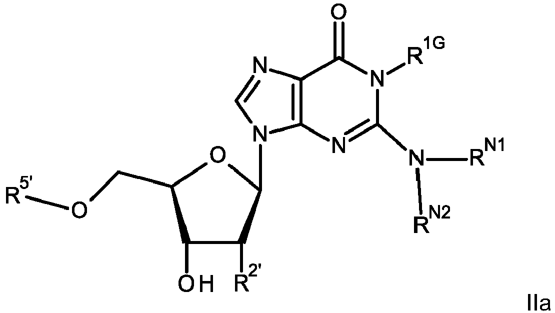
- wherein R^{N1}, R^{N2}, R^{1A}, R^{1G}, R^{2'} and R^{5'} can have the meaning indicated above,
- and wherein R^{2'} is selected from H, OH, O-methyl, ribosyl and 5"phosphoribosyl, and R^{5'} is selected from H, PO₃²⁻ and methyl.

In certain embodiments of any of the nucleoside derivative compounds disclosed herein as MR1 ligands, the MR1 ligand compound is described by
a. formula (I) wherein R² is S-methyl and R^{N1} and R^{N2} are both H;
b. formula (I) wherein R² is methyl and R^{N1} and R^{N2} are both H; or
c. formula (Ia) wherein R^{1A} is methyl, R² is H and R^{N3} is H; or
d. formula (Ia) wherein R^{1A} is methyl, R² is methyl and R^{N3} is H; or
e. formula (Ia) wherein R^{1A} is methyl, R² is S-methyl and R^{N3} is H; or
f. formula (I) wherein R² is H, one of R^{N1} and R^{N2} is selected from H and methyl, and the other one of R^{N1} and R^{N2} is selected from methyl, 3-methylbut(2)enyl (isopent-2-enyl), 3-hydroxymethylbut(2)enyl (hydroxyisopent-2-enyl) and threonylcarbamoyl; or
g. formula (I) wherein R² is S-methyl and one of R^{N1} and R^{N2} is selected from H and methyl, and the other one of R^{N1} and R^{N2} is selected from methyl, 3-methylbut(2)enyl (isopent-2-enyl), 3-hydroxymethylbut(2)enyl (hydroxyisopent-2-enyl) and threonylcarbamoyl;
h. formula (I) wherein R² is methyl and one of R^{N1} and R^{N2} is selected from H and methyl, and the other one of R^{N1} and R^{N2} is selected from methyl, 3-methylbut(2)enyl (isopent-2-enyl), 3-hydroxymethylbut(2)enyl (hydroxyisopent-2-enyl) and threonylcarbamoyl;
i. formula (II) wherein R^{1G} is methyl, R⁷ is an unbound electron pair and R^{N1} and R^{N2} are both H; or
j. formula (II) wherein R^{1G} is methyl, R⁷ is methyl and R^{N1} and R^{N2} are both H; or
k. formula (II) wherein R^{1G} is H, R⁷ is methyl and R^{N1} and R^{N2} are both H; or
l. formula (II) wherein R^{1G} is methyl, R⁷ is an unbound electron pair and one of R^{N1} and R^{N2} is selected from H and methyl, and the other one of R^{N1} and R^{N2} is selected from methyl, 3-methylbut(2)enyl (isopent-2-enyl), 3-hydroxymethylbut(2)enyl (hydroxyisopent-2-enyl) and threonylcarbamoyl; or
m. formula (II) wherein R^{1G} is methyl, R⁷ is methyl and one of R^{N1} and R^{N2} is selected from H and methyl, and the other one of R^{N1} and R^{N2} is selected from methyl, 3-methylbut(2)enyl (isopent-2-enyl), 3-hydroxymethylbut(2)enyl (hydroxyisopent-2-enyl) and threonylcarbamoyl; or
n. formula (II) wherein R^{1G} is H, R⁷ is methyl and one of R^{N1} and R^{N2} is selected from H and methyl, and the other one of R^{N1} and R^{N2} is selected from methyl, 3-methylbut(2)enyl (isopent-2-enyl), 3-hydroxymethylbut(2)enyl (hydroxyisopent-2-enyl) and threonylcarbamoyl; or
o. formulas (I-IM or II-IM), with R^{IM} selected from H, CH₂COCₙH₍₂ₙ₊₁₎ with n from 3 to 7 (part. n=5), and CH₂CO(CH₂)ₘCOO⁻ with m from 3 to 9 (part. 7) 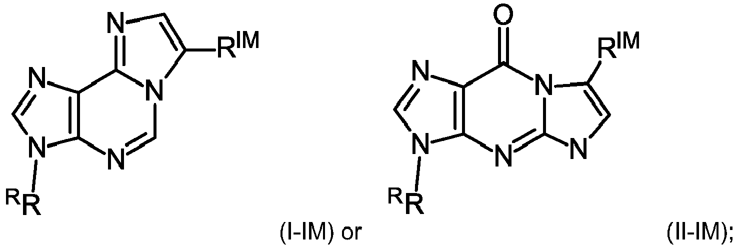 or
p. formulas (II-c), (II-d), (II-e) (II-f) 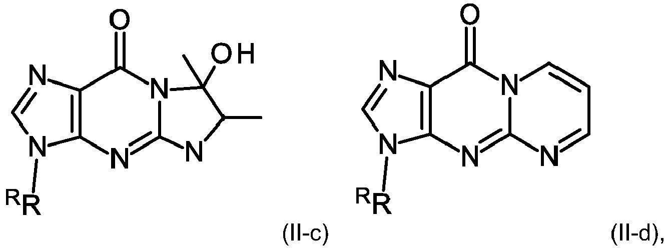 or 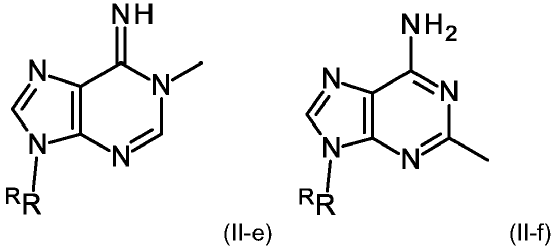
q. formulas (II-g) or (II-h), wherein R² is selected from H, methyl and S-methyl: 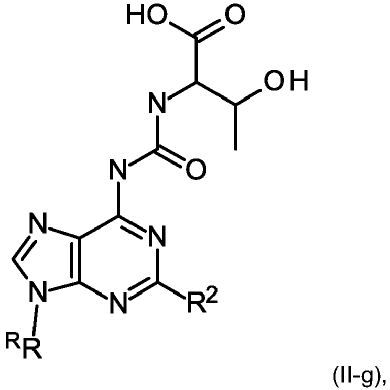 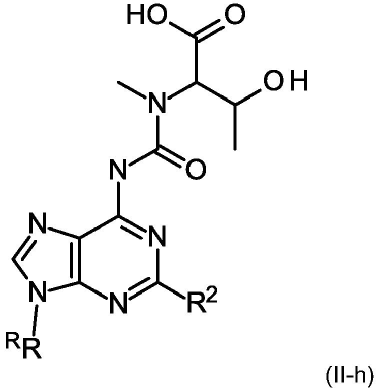
r. formulas (II-i) (II-j), wherein R^{N1} is selected from H and methyl and wherein R² is selected from H, methyl and S-methyl: 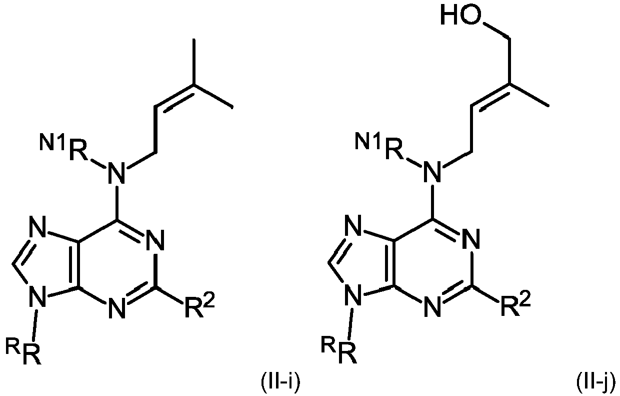
s. formula (III), wherein R^{3U} is H and R^{5U} is methyl,
t. formula (III), wherein R^{3U} is methyl and R^{5U} is H,
u. formula (III), wherein R^{3U} and R^{5U} are both methyl,
v. formula (IV), wherein R^{3C} and R^{5C} are H, one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl;
w. formula (IV), wherein R^{3C} is methyl and R^{5C} is H, one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl; or
x. formula (IV), wherein R^{3C} is H and R^{5C} is methyl, one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl;
and wherein in each formula, R^{R} has the meaning as indicated in claim 1 or 2.

In certain embodiments of any of the nucleoside derivative compounds disclosed herein as MR1 ligands, the MR1 ligand compound is selected from
a. 1-methyladenosine
b. 2-methyladenosine
c. 2'-O-methyladenosine
d. N6,N6-dimethyladenosine
e. N6-threonylcarbamoyladenosine
f. N6-isopent-2-enyladenosine
g. N6-(cis-hydroxyisopent-2-enyl) adenosine
h. 2-methylthio-N6-(cis-hydroxyisopent-2-enyl) adenosine
i. 2-methylthio-N6-isopent-2-enyladenosine
j. N6-methyl-N6-threonylcarbamoyladenosine
k. 2'-O-ribosyladenosinephosphate
l. (3*Z*,5*E*)-6-((9*H*-purin-6-yl) amino) hexa-1,3,5-triene-1,1,3-tricarbaldehyde
m. N6-(3-oxo-1-propenyl)-2'-deoxyadenosine
n. 8-(9-((2*R*,4*S*,5*R*)-4-hydroxy-5-(hydroxymethyl) tetrahydrofuran-2-yl)-9*H-*purin-6-yl)-2-oxa-8-azabicyclo [3.3.1] nona-3,6-diene-4,6-dicarbaldehyde
o. 1-(3-((2*R*,4*S*,5*R*)-4-hydroxy-5-(hydroxymethyl) tetrahydrofuran-2-yl)-3*H-*imidazo[2,1-*i*] purin-7-yl) heptan-2-one
p. 1-methylguanosine
q. N2-methylguanosine
r. 2'-O-methylguanosine
s. N2,N2-dimethylguanosine
t. 3-((2*R*,3*R*,4*S*,5*R*)-3,4-dihydroxy-5-(hydroxymethyl)tetrahydrofuran-2-yl)-6,7-dihydroxy-7-methyl-6,7-dihydro-3*H*-imidazo[1,2-a]purin-9(5*H*)-one
u. 2-((6-oxo-6,7-dihydro-1*H*-purin-2-yl)amino)propanoate
v. 3-((2*R*,5*R*)-4-hydroxy-5-(hydroxymethyl) tetrahydrofuran-2-yl)-7-(2-oxoheptyl)-3*H*-imidazo[1,2-*a*]purin-9(5*H*)-one
w. 3-(2-deoxy-β-D-erythro-pentofuranosyl)pyrimido[1,2-a]purin-10(3H)-one
x. N2-oxopropenyl-deoxyguanosine
y. 3-((2*R*,4*S*,5*R*)-4-hydroxy-5-(hydroxymethyl)tetrahydrofuran-2-yl)-12-oxo-5,6,10,12-tetrahydro-3*H*-6,10-methano[1,3,5]oxadiazocino[5,4-*a*]purine-9-carbaldehyde
z. 2'-*O*-methylcytidine
aa. 3-methyluridine
bb. 5-methyluridine
cc. 3,2'-O-dimethyluridine
dd. 6-((2*R*,4*S*,5*R*)-4-hydroxy-5-(hydroxymethyl)tetrahydrofuran-2-yl)-3-(2-oxoheptyl)-1,8a-dihydroimidazo[1,2-c]pyrimidin-5(6*H*)-one
ee. N4-(3-oxo-1-propenyl)-2'-deoxycytidine
ff. 8-(1-((2*R*,4*S*,5*R*)-4-hydroxy-5-(hydroxymethyl)tetrahydrofuran-2-yl)-2-oxo-1,2-dihydropyrimidin-4-yl)-2-oxa-8-azabicyclo[3.3.1]nona-3,6-diene-4,6 dicarbaldehyde
gg. (2*R*,3*S*,4*R*,5*R*)-2-(hydroxymethyl)-5-(6-(methylthio)-9*H*-purin-9-yl)tetrahydrofuran-3,4-diol

The invention further encompasses variations of the modifications shown herein that can be obtained by modifying the moieties attached to the nucleoside core structure by adding amine functions, hydroxy functions, carboxylic acid or carboxylic acid ester moieties or halogens. Any of the compounds showing an alkyl moiety above may be modified to show an aminoalkyl, hydroxyalkyl or haloalkyl as defined above. Mixed modifications are possible. The assays shown herein provide a solid basis for testing whether such modifications are capable of binding to MR1 and of triggering or inhibiting a MR1T cell response.

In a general aspect, the invention relates to a method for modulating, particularly in vitro, an interaction between an MR1 polypeptide and an MR1-specific TCR molecule. The method according to this aspect comprises contacting the MR1 polypeptide with an MR1 ligand compound as specified above.

According to one aspect of the invention, the method for modulating an interaction between an MR1 polypeptide and an MR1-specific TCR molecule can be employed as part of a method for the identification and isolation of T cells reactive to the MR1 ligand compound as presented on MR1.

According to another aspect of the invention, the method for modulating an interaction between an MR1 polypeptide and an MR1 -specific TCR molecule can be employed as part of a method of diagnosis to classify metabolically altered cells, including but not limited to tumor cells, according to the presence of the compounds, wherein a sample obtained from a patient is analyzed with regard to the presence of an MR1 ligand compound as identified herein.

Yet another aspect of the invention relates to the use of a compound as specified as an MR1 ligand compound above, for use in modulating an MR1-dependent immune response for prophylaxis or treatment of a disease associated with an aberrant or absent MR1-specific T cell response, particularly in treatment of cancer characterized by tumor cells expressing MR1. In certain embodiments of the use in modulating an MR1-dependent immune response for prophylaxis or treatment of a disease associated with an aberrant or absent MR1-specific T cell response, the compound is co-administered with an anticancer drug, and/or checkpoint modulator agent.

In certain embodiments, the compound is co-administered with a preparation comprising (transgenic) MR1-reactive T cells and/or a polynucleotide expression vector encoding MR1.

The invention further relates to a method for identification of a T cell reactive to a MR1 ligand compound as specified herein. The method according to this aspect of the invention comprises the steps of providing a preparation of T cells reactive to /capable of specifically recognizing MR1-expressing cells; contacting this preparation of T cells with a complex comprising isolated MR1 associated to said compound as specified herein; and isolating a T cell that is specifically reactive to said MR1 ligand compound in an isolation step.

Another aspect of the invention is the use of a compound which is capable of inhibiting decarbonylation inside a cell for increasing an MR1-dependent immune response selected from disulfiram, daidzin, ellagic acid, oleanoic acid, erythro-9-(2-hydroxy-3-nonyl) adenine (EHNA), and mycophenolic acid for prophylaxis or therapy of a disease associated with aberrant or lacking MR1 expression, particularly treatment or prevention of recurrence of cancer disease associated with tumor cells expressing MR1.

The compounds identified herein can be employed in methods pertaining to:
- modulating an interaction between an MR1 (major histocompatibility complex class I-related gene protein 1) polypeptide and an MR1-specific T cell receptor molecule, wherein the MR1 polypeptide is contacted with the MR1 ligand compound;
- generating a panel of MR1 multimeric reagents to use for identification and sorting of T cells reacting to MR1 ligand compounds presented on non-polymorphic MHC I-related MR1 antigen-presenting molecules;
- identifying a TCR gene specifically reactive to said compounds to be used in a personalized cellular immunotherapy. This may be achieved by providing a preparation of tumor cells isolated from a patient, and submitting it to mass-spectrometry-based determination for assessing the presence and identity of the compounds.

In certain embodiments identifying a TCR gene specifically reactive to the compounds identified herein for use in a personalized cellular immunotherapy can be effected by the following general sequence of processes: 1) compound detection in a tumor biopsy (the tumor is classified based on the TAA presence) and then 2) guided TCR-mediated cellular immunotherapy based on the TCR best reacting to the identified TAA in complex with MR1. Another aspect of the invention relates to the use of pharmaceutical drugs capable of inhibiting enzymatic decarbonylation and thus facilitating the accumulation or disposal of MR1 ligand compounds (MR1T cell-stimulatory compounds) as identified herein.

The following compound classes and specific drugs identified as part of the compound class are possible combination partners to the MR1 TAAs in vaccination-plus-drug therapeutic approaches, or MR1-T-cell-transfer-plus-drug therapeutic approaches:
**Inhibitors of aldehyde dehydrogenases**, particularly any one of the inhibitors of aldehyde dehydrogenases selected from the group comprised of Ampal, Benomyl, Citral, Chloral hydrate, Chlorpropamide analogs (NPI-1 and API-1), Coprine, Cyanamide, **Daidzin**, CVT-10216, 4-(Diethylamino)benzaldehyde (DEAB), **Disulfiram**, Gossypol, Molinate, Nitroglycerin, Pargyline,
In one alternative of this aspect of the invention, disulfiram is provided for treatment of cancer disease associated with tumor cells expressing MR1, or prevention of recurrence of such tumor cells. **Disulfiram** (1,1',1",1"'-[disulfanediylbis(carbonothioylnitrilo)] tetraethane, CAS No. 97-77-8) 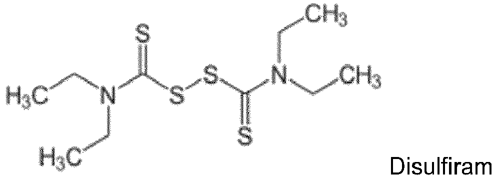 is a well-characterized small molecule drug that has been used for more than 50 years for the treatment of alcoholism in humans. Bulk chemical synthesis procedures are known; extensive pharmacological and toxicological data on the compound exist.

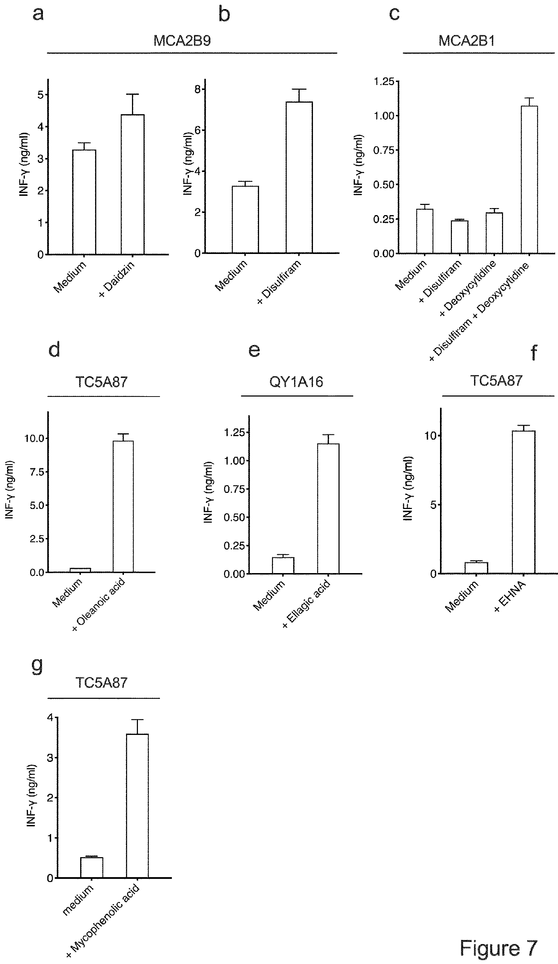

Studies have shown a degree of effectiveness against fungi (S. Khan et al., Jp. J. Med. Mycol (2007), 48, 109-113), protozoa (T. Nash and W.G. Rice, Antimicrob. Agents Chemother. (1998) 42, 1488-1492) and bacteria (MRSA, M. Phillips et al., Antimicrob. Agents Chemother. (1991), 35, 785 - 787) for disulfiram. The results shown in Fig. 7 support the compound's usefulness in administration as part of a MR1T targeted treatment (i.e. a prophylaxis or therapy of a disease associated with aberrant or lacking MR1 expression, particularly treatment or prevention of recurrence of cancer disease associated with tumor cells expressing MR1).

In another alternative of this aspect of the invention, daidzin is provided for treatment of cancer disease associated with tumor cells expressing MR1, or prevention of recurrence of such tumor cells. **Daidzin** is an isoflavone phytochemical (CAS No. 552-66-9). The results shown in Fig. 7 support the compound's usefulness in administration as part of a MR1T targeted treatment.

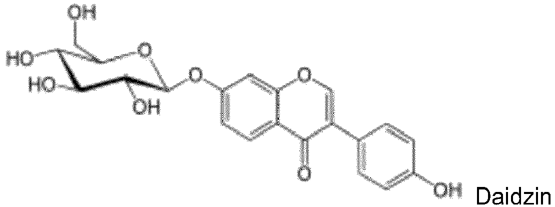

In yet another alternative of this aspect of the invention, **ellagic acid** (CAS No 476-66-4) and/or **oleanoic acid** (CAS No. 508-02-1) are provided for treatment of cancer disease associated with tumor cells expressing MR1, or prevention of recurrence of such tumor cells. The results shown in Fig. 7 support the compounds' usefulness in administration as part of a MR1T targeted treatment (i.e. a prophylaxis or therapy of a disease associated with aberrant or lacking MR1 expression, particularly treatment or prevention of recurrence of cancer disease associated with tumor cells expressing MR1).

Another aspect of the invention relates to the use of pharmaceutical drugs which facilitate the accumulation of nucleobases, thus increasing antigen availability. These drugs include, but are not limited to, the inhibitors of adenosine deaminase 1 (ADA1), **erythro-9-(2-hydroxy-3-nonyl) adenine (EHNA)**, and inhibitors of both ADA1 and ADA2, pentostatin (CAS No. 53910-25-1) and 1-deazaadenosine. They also include inhibitors of dehydrogenase 1 (IMPDH1) such as, but not limited to, **mycophenolic acid** (CAS No. 24280-93-1). The results shown in Fig. 7 support the compounds' usefulness in administration as part of a MR1T targeted treatment.

A further aspect of the invention relates to the MR1 ligand compounds identified herein according to the first aspect of the invention, particularly an MR1 ligand identified by any one of formulas (I), (Ia), (II), (III) or (IV), for use as a vaccine to elicit or boost an MR1T cell response, particularly in treatment or prevention of recurrence of cancer. In particular embodiments, the MR1 ligand compounds identified herein may be employed as a combination medicament to stimulate and augmenting antitumor activity of MR1T cells used in cellular therapy, and/or may be used in combination with immunostimulatory compounds and/or checkpoint modulator agents.

The MR1 ligand compounds identified herein may also be used as a vaccine in a subject without overt disease, but with a predisposition to develop such disease. Such as a human subject can be treated with preventative vaccination in advance of each of the maladies described herein.

Likewise, an alternative of this aspect of the invention relates to the MR1 ligand compounds identified herein according to the first aspect of the invention, particularly an MR1 ligand identified by any one of formulas (I), (Ia), (II), (III) or (IV), for use in a combination medicament in combination with oncotherapeutic agents.

One important advantage of the methods and compounds provided herein relates to the ability to test and provide TAAs to patients regardless of their MHC haplotype. TAA are the targets of clinically relevant anti-tumor immune response in cancer patients. Nevertheless, the majority of the so far identified TAAs are peptides presented by polymorphic MHC molecules. The extreme polymorphism of MHC genes limits the TAA targeting to those patients expressing certain MHC alleles. Targeting TAAs bound to MR1 non-polymorphic antigen-presenting molecules overcomes this constraint and is applicable to all patients bearing tumors expressing MR1. In addition, because tumor cells may express different non-peptidic TAAs, this strategy provides the possibility of targeting multiple antigens displayed by the same tumor cells, thus minimizing the potential occurrence of tumor escape-variants under selective immune pressure. Therefore, the identification of MR1-presented TAAs, matched with the specific MR1-restricted TCRs recognizing these antigens, has important implications for cancer immunotherapy.

In certain embodiments, the MR1 ligand compounds identified herein are provided for use in combination with an immune checkpoint modulator, particularly in combination with an immune checkpoint inhibitor agent.

In certain embodiments, the immune checkpoint inhibitor agent is ipilimumab (Yervoy; CAS No. 477202-00-9).

In certain embodiments, the immune checkpoint inhibitor agent is an inhibitor of interaction of programmed cell death protein 1 (PD-1) with its receptor PD-L1. In certain embodiments, the immune checkpoint inhibitor agent is selected from the clinically available antibody drugs nivolumab (Bristol-Myers Squibb; CAS No. 946414-94-4), pembrolizumab (Merck Inc.; CAS No. 1374853-91-4), pidilizumab (CAS No. 1036730-42-3), atezolizumab (Roche AG; CAS No. 1380723-44-3), and avelumab (Merck KGaA; CAS No. 1537032-82-8).

In certain embodiments, the MR1 ligand compounds identified herein are provided for use in combination, wherein any of the MR1 ligand compounds identified herein is a first combination partner, and
a) a modified T cell reactive to an MR1 molecule presenting the MR1 ligand compound and/or a nucleic acid expression vector encoding MR1 is a second combination partner, and
b) an immune checkpoint modulator, particularly an immune checkpoint inhibitor agent is a third combination partner.

Such combination is likely to be in a form where the combination partners are applied at different times and in different administration forms during treatment.

The identification of the compounds identified herein, which are presented by tumor cells in each tumor patient, represents a novel method to classify tumors. This classification is of relevance to select the appropriate MR1T-derived TCR to be used in a personalized TCR gene therapy.

Tumor patients can be also vaccinated with selected compounds previously detected in the tumor cells from the same patient. This treatment will have the goal of eliciting and/or stimulating MR1T cells specific for the compound and thus capable of recognizing and killing tumor cells.

Some of the MR1 ligand compounds described herein are also present in tissues of patients with autoimmune and metabolic diseases, including rheumatoid arthritis, systemic lupus erythematosus, type I diabetes, atherosclerosis, inflammatory bowel disease and multiple sclerosis. In general, disease with abnormal generation of Reactive Oxygen Species (ROS) are characterized by an accumulation of the MR1-binding compounds described below. In these cases, autoreactive MR1T cells that are stimulated during these diseases represent large T cell populations that may be inhibited in a therapeutic setting.

The inventors predict that appropriate therapeutic intervention forms can be found using those described for tumor immunotherapy as a template. The types of diseases which could be similarly treated because of the accumulation of the same types of MR1 ligands may thus be expanded.

Yet another aspect of the invention relates to the detection of MR1 ligand compounds identified herein as part of a method of disease classification, wherein the presence of at least one of the MR1 ligand compounds according to the invention is identified in samples from patients. In case of cancer patients, the identification of these compounds extracted from fresh tumor samples is considered a diagnostic marker. In certain embodiments, the TAA MR1 ligand compounds identified herein can be used for guiding cellular immunotherapy and also vaccination in combination with other therapeutic interventions, in other words, once the MR1 ligand is identified as present in the tumor, patients could be treated with personalized immunotherapy interventions including i) administration of pharmaceutical drugs capable of facilitating the accumulation of MR1T cell-stimulatory compounds as identified herein, ii) administration of selected TAA MR1 ligand compounds, iii) MR1 TCR-mediated cell therapy alone or in combination with other oncotherapeutic agents.

The invention also relates to a research method directed at the identification of T cells reactive to MR1-expressing cells. This encompasses a method to isolate from peripheral blood of normal donors or from patients suffering from cancer, metabolic or autoimmune diseases, MR1-restricted T cells sorted by using compound-loaded onto MR1 multimeric molecules. The cell source encompasses, but is not limited to, T cells isolated from tissue biopsies.

The invention also relates to the possibility to readily identify the TCR gene and protein sequences expressed by the above-mentioned T cells.

In certain aspects, the current invention is centered on the identification of novel classes of compounds which bind to non-polymorphic MR1 molecules. Some of the compounds identified herein modulate the surface expression of MR1. Some of the compounds identified herein (not necessarily the same compounds as those found to modulate the surface expression of MR1) are antigens, stimulating specific human T cells restricted to MR1. Some of the compounds identified herein were isolated from tumor cells, they were purified, identified and synthetic analogs were produced. The compounds showing antigenic activity, when presented in association with MR1 molecules, stimulate a population of human T cells discovered by the inventors and termed MR1T cells. Applications of this invention encompass, but are not limited to, the following methods.
i) a method to stimulate compound-specific T cells, and induce a prophylactic immune response,
ii) a method to stimulate compound-specific T cells, and induce a therapeutic immune response,
iii) a method for the identification and isolation of T cells reactive to the compounds,
iv) a method to modulate (increasing or decreasing) the quantity of defined compounds in the cells or presented by the cells,
v) a method to classify metabolically altered cells, including but not limited to tumor cells, according to the presence of the compounds.

Thus, in certain aspects and embodiments, the invention relates to the use of the MR1-associated compounds identified herein, for guiding personalized intervention of immunotherapy, of cellular immunotherapy, of vaccination strategies in people at risk and for diagnostics tests of several diseases, including cancer.

### Medical treatment, Dosage Forms and Salts

Similarly, within the scope of the present invention is a method or treating a condition associated with a lack of MR1-specific T cell responses, or with too much of an MR1-specific T cell response, in a patient in need thereof, comprising administering to the patient a compound as specified in detail above.

Similarly, a dosage form for the prevention or treatment of a condition associated with a lack of MR1-specific T cell responses, or with too much of an MR1-specific T cell response is provided, comprising a non-agonist ligand or antisense molecule according to any of the above aspects or embodiments of the invention.

The skilled person is aware that any specifically mentioned drug may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

Topical administration is also within the scope of the advantageous uses of the invention. The skilled artisan is aware of a broad range of possible recipes for providing topical formulations, as exemplified by the content of Benson and Watkinson (Eds.), Topical and Transdermal Drug Delivery: Principles and Practice (1st Edition, Wiley 2011, ISBN-13: 978-0470450291); and Guy and Handcraft: Transdermal Drug Delivery Systems: Revised and Expanded (2nd Ed., CRC Press 2002, ISBN-13: 978-0824708610); Osborne and Amann (Eds.): Topical Drug Delivery Formulations (1st Ed. CRC Press 1989; ISBN-13: 978-0824781835).

### Pharmaceutical Compositions and Administration

Another aspect of the invention relates to a pharmaceutical composition comprising a compound as specified herein in the context of the present invention, or a pharmaceutically acceptable salt thereof, and a pharmaceutically acceptable carrier. In further embodiments, the composition comprises at least two pharmaceutically acceptable carriers, such as those described herein.

In certain embodiments of the invention, the compound of the present invention is typically formulated into pharmaceutical dosage forms to provide an easily controllable dosage of the drug and to give the patient an elegant product, which is easy to handle.

In embodiments of the invention relating to topical uses of the compounds of the invention, the pharmaceutical composition is formulated in a way that is suitable for topical administration such as aqueous solutions, suspensions, ointments, creams, gels or sprayable formulations, e.g., for delivery by aerosol or the like, comprising the active ingredient together with one or more of solubilizers, stabilizers, tonicity enhancing agents, buffers and preservatives that are known to those skilled in the art.

The pharmaceutical composition can be formulated for oral administration, parenteral administration, or rectal administration. In addition, the pharmaceutical compositions of the present invention can be made up in a solid form (including without limitation capsules, tablets, pills, granules, powders or suppositories), or in a liquid form (including without limitation solutions, suspensions or emulsions).

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Description of the Figures

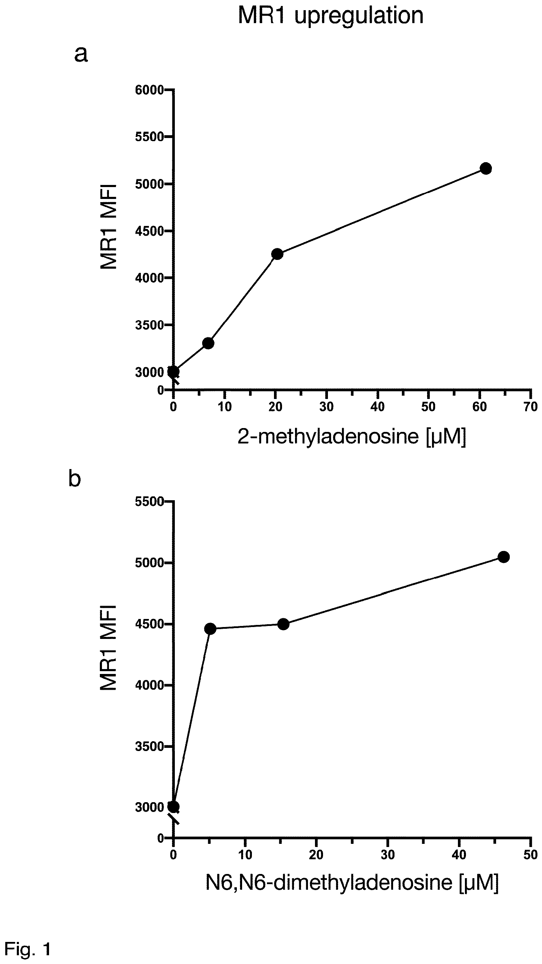

- Fig. 1: shows an example of cell surface MR1 upregulation on APCs by two compounds. a) 2-Methyladenosine and b) N6,N6-dimethyladenosine were used at the three indicated concentrations. Expression of MR1 on THP1-MR1 cells was assessed by flow cytometry and is shown as median fluorescence intensity (MFI) compared to the baseline expression on the cells incubated with vehicle only.

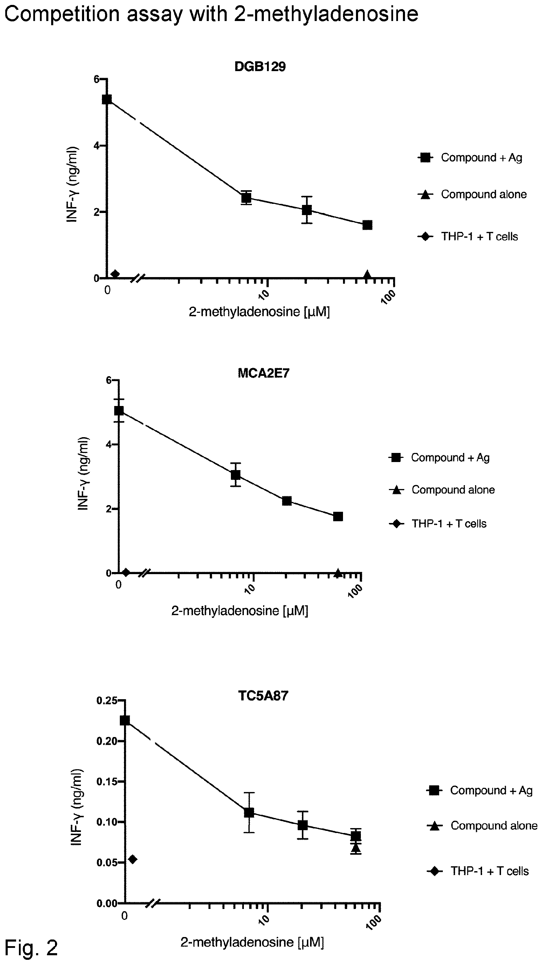

- Fig. 2: shows an example of a competition assay for stimulation of three MR1T cell clones. 2-Methyladenosine was used with THP-1 cells at the 3 indicated concentrations, before adding the optimal dose of antigen for each of the illustrated T cell clones (DGB129, MCA2E7 and TC5A87). The response of the T cell clones is shown as mean ± sd of IFN-γ release. The T cell response in the presence of antigen alone, of compound alone or of THP-1 cells only are also shown

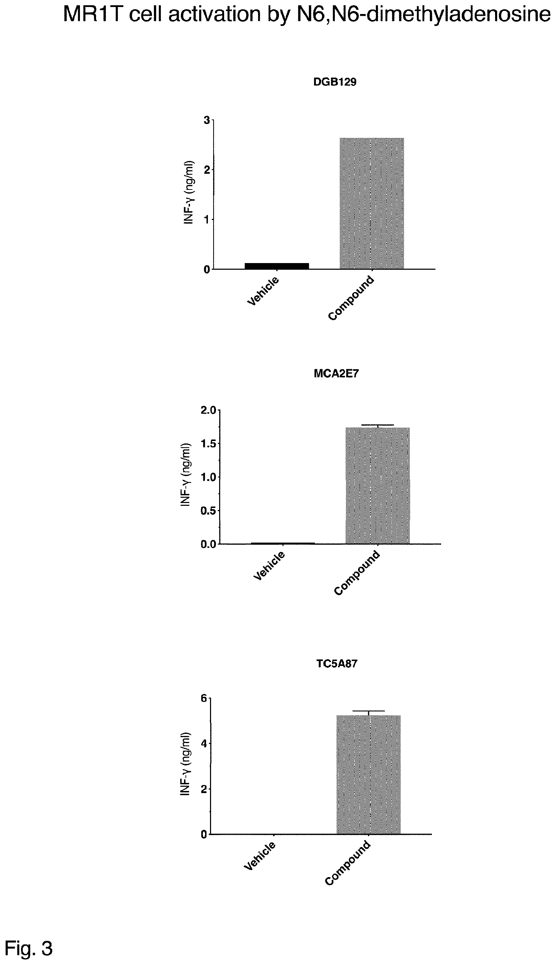

- Fig. 3: shows an example of compound-specific MR1T cell activation. The response of three MR1T cell clones (DGB129, MCA2E7 and TC5A87) to THP-1 cells incubated with the compound N6,N6-dimethyladenosine is shown as mean ± sd of the IFN-γ released after overnight stimulation. The response of the T cells to THP-1 cells and compound vehicle are shown as control.

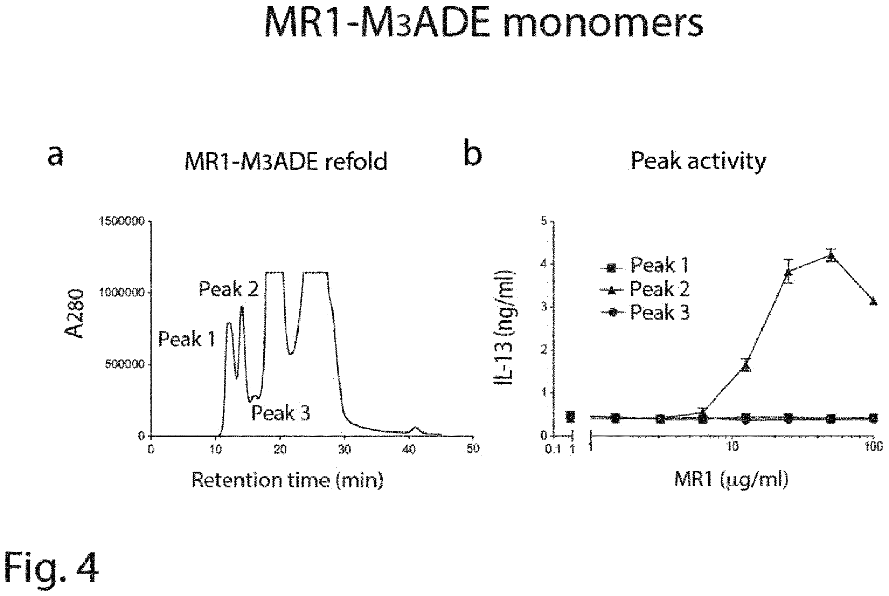

- Fig. 4: shows the generation of M₃ADE-loaded MR1 monomers: a) Gel filtration chromatography purification of MR1 protein refolded in the presence of M₃ADE. Absorption at 280 nm and retention time (min) are shown on the y- and x-axis, respectively. The indicated peaks 1, 2, and 3 were collected and b) were used to activate DGB129 cells in a plate-bound assay. IL-13 released by the T cells is shown as mean ± sd of duplicates.

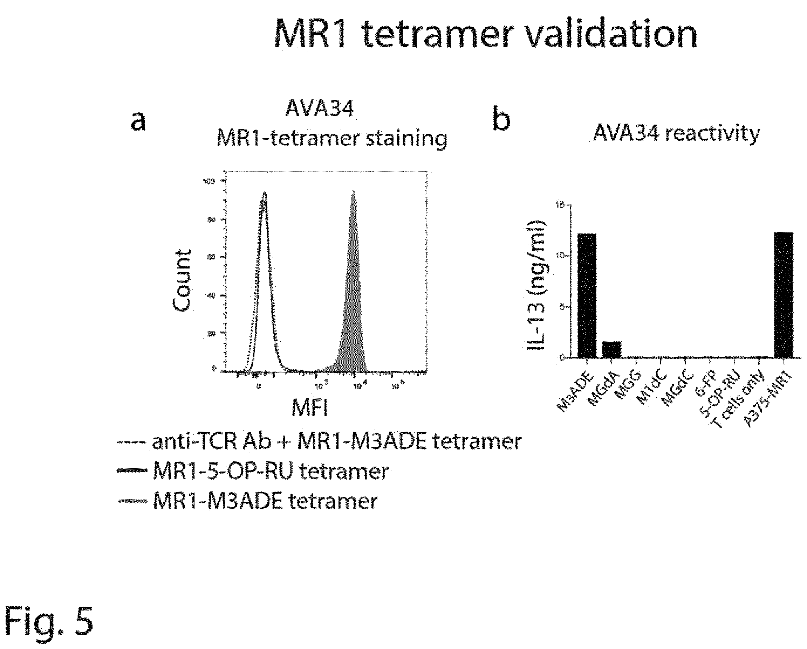

- Fig. 5: shows the validation of MR1-M₃ADE tetramer staining. The MR1T cell clone AVA34 was generated by two rounds of FACS sorting of CD3⁺, MR1-M₃ADE tetramer⁺ cells from PBMCs, followed by PHA stimulation and cloning by limiting dilution of the M₃ADE-reactive T cells. a) Histogram overlay showing the staining results of AVA34 cells with MR1-M₃ADE, and MR1-5-OP-RU tetramers as well as with MR1-M₃ADE tetramer after incubation with anti-TCR mAb specific for Vβ8 (JR2, 1µg/ml), which prevented M₃ADE tetramer staining. b) Bar chart showing IL-13 release by AVA34 cells toward THP-1 cells in the presence of M₃ADE. Low levels of IL-13 are released in the presence of MGdA, but not of the other indicated compounds. A375-MR1 cells were used as positive control and T cells alone as negative control.

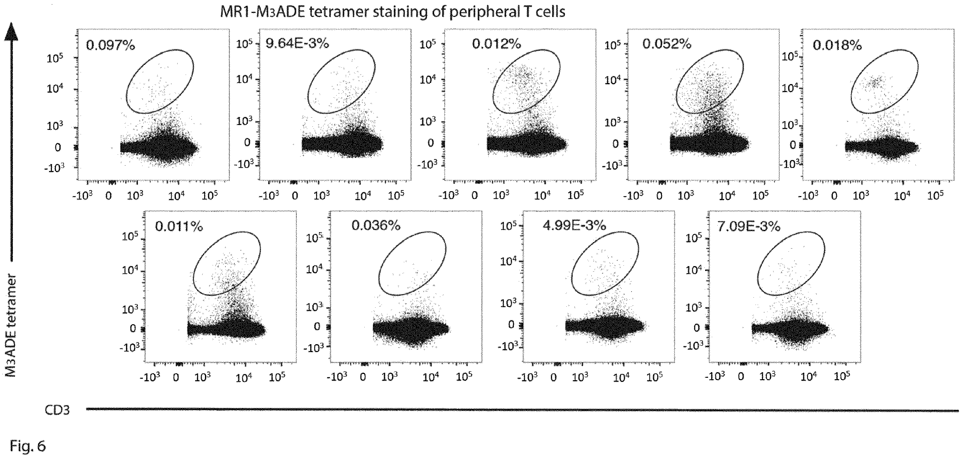

- Fig. 6: shows the *ex vivo* frequency of MR1-M₃ADE tetramer⁺ MR1T cells in the blood of healthy donors. Dot plots of PBMCs from 9 healthy donors co-stained with MR1-M₃ADE tetramer and anti-CD3 (UCHT1). Each plot represents an individual donor. Cells were gated as live CD3⁺, CD14⁻, CD19⁻ single cells and numbers indicate the percentage of MR1-M₃ADE tetramer⁺ MR1T cells within the oval gate.
- Fig. 7: shows the treatment of APCs with drugs that induce accumulation of carbonyl-containing molecules stimulate MR1T cells. a) Daidzin and b) Disulfiram increase the response of MCA2B9 MR1T clone. c) Disulfiram induces the stimulation of the MCA2B1 MR1T clone when exogenous deoxycytidine is co-administered. d) Oleanoic acid induces the stimulation of the TC5A87 MR1T clone, e) Ellagic acid induces the stimulation of the QY1A16 MR1T clone, f) EHNA induces the stimulation of the TC5A87 MR1T clone, and g) Mycophenolic acid induces the stimulation of the TC5A87 MR1T clone. APCs (THP-1 cells) were incubated 18 h with individual drugs before addition of MR1T cells and of deoxycytidine (only in panel c). Drugs did not induce stimulation of MR1T cells in the absence of APCs. The responses of the T cell clones are expressed as IFN-γ release (mean ± sd) of triplicates.

**Table 1. List of exemplary active compounds with structures and references.**

| | | |
|---|---|---|
| Full name | 1-methyladenosine | 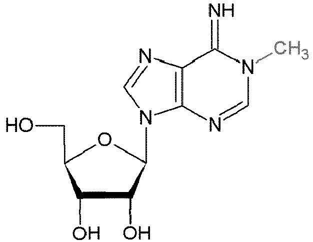 |
| Short name | m1A | |
| CAS Number | 15763-06-1 | |
| Molecular Formula | C₁₁H₁₆N₆O₄ | |
| Molecular Weight | 281.27 | |
| Ref. | Ishiwata, Itoh et al. 1995 | |
| | Seidel, Brunner et al. 2006 | |
| Company, Cat. | Cayman Chemical, 16937 | 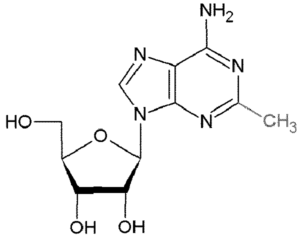 |
| Full name | 2-methyladenosine | |
| Short name | m2A | |
| CAS Number | 16526-56-0 | |
| Molecular Formula | C₁₁H₁₅N₅O₄ | |
| Molecular Weight | 281.27 | |
| Ref. | NA | |
| Company, Cat. | Santa Cruz, sc-500888 | |
| Full name | 2'-O-methyladenosine | 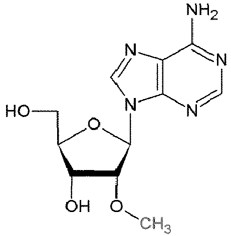 |
| Short name | Am | |
| CAS Number | 2140-79-6 | |
| Molecular Formula | C₁₁H₁₅N₅O₄ | |
| Molecular Weight | 281.27 | |
| Ref. | NA | |
| Company, Cat. | Cayman Chemical, 16936 | |
| Full name | N6,N6-dimethyladenosine | 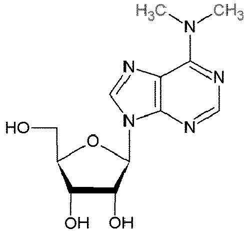 |
| Short name | m6,6A or m6,2A | |
| CAS Number | 2620-62-4 | |
| Molecular Formula | C₁₂H₁₇N₅O₄ | |
| Molecular Weight | 295.29 | |
| Ref. | NA | |
| Company, Cat. | NA | |
| Full name | N6-threonylcarbamoyladenosine | 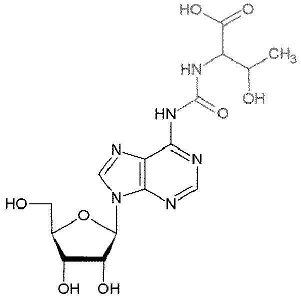 |
| Short name | t6A | |
| CAS Number | 24719-82-2 | |
| Molecular Formula | C₁₅H₂₀N₆O₈ | |
| Molecular Weight | 412.35 | |
| Ref. | NA | |
| Company, Cat. | Toronto Research Chemicals, T405560 | |
| Full name | N6-isopent-2-enyladenosine | |
| Short name | i6A | 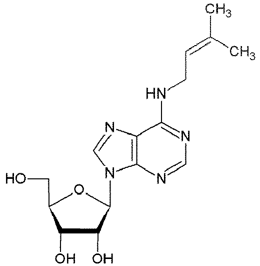 |
| CAS Number | 7724-76-7 | |
| Molecular Formula | C₁₅H₂₁N₅O₄ | |
| Molecular Weight | 335.36 | |
| Ref. | NA | |
| Company, Cat. | Toronto Research Chemicals, I821840 | |
| Full name | N6-(cis-hydroxyisopent-2-enyl) adenosine | 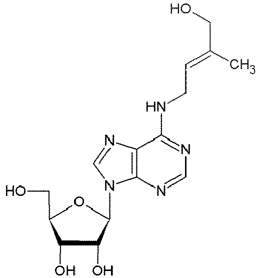 |
| Short name | io6A | |
| CAS Number | 15896-46-5 | |
| Molecular Formula | C₁₅H₂₁N₅O₅ | |
| Molecular Weight | 351.36 | |
| Ref. | NA | |
| Company, Cat. | NA | |
| | | |
| Full name | 2-methylthio-N6-(cis-hydroxyisopent-2-enyl) adenosine | 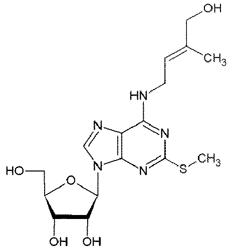 |
| Short name | ms2io6A | |
| CAS Number | 52049-48-6 | |
| Molecular Formula | C₁₆H₂₃N₅O₅S | |
| Molecular Weight | 397.45 | |
| Ref. | NA | |
| Company, Cat. | Toronto Research Chemicals, M330525 | |
| Full name | 2-methylthio-N6-isopent-2-enyladenosine | 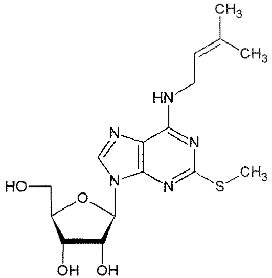 |
| Short name | ms2i6A | |
| CAS Number | 20859-00-1 | |
| Molecular Formula | C₁₆H₂₃N₅O₄S | |
| Molecular Weight | 381.45 | |
| Ref. | NA | |
| Company, Cat. | Toronto Research Chemicals, M330675 | |
| Full name | N6-methyl-N6-threonylcarbamoyladenosine | 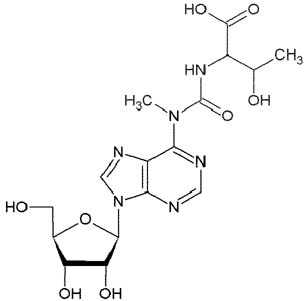 |
| Short name | m6t6A | |
| CAS Number | 39667-81-7 | |
| Molecular Formula | C₁₆H₂₂N₆O₃ | |
| Molecular Weight | 426.38 | |
| Ref. | NA | |
| Company, Cat. | NA | |
| Full name | 2'-O-ribosyladenosine (phosphate) | 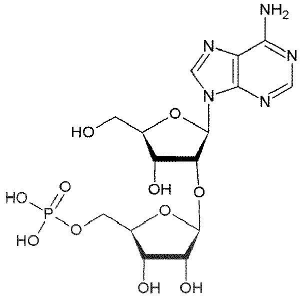 |
| Short name | Ar(p) | |
| CAS Number | 28050-13-7 | |
| Molecular Formula | C₁₅H₂₂N₅O₁₁P₁ | |
| Molecular Weight | 479.34 | |
| Ref. | NA | |
| Company, Cat. | NA | |
| | | |
| Full name | (3*Z*,5*E*)-6-((9*H*-purin-6-yl) amino) hexa-1,3,5-triene-1,1,3-tricarbaldehyde (synthetic adduct) | 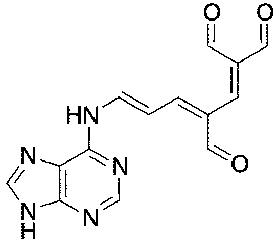 |
| Short name | M₃ADE | |
| CAS Number | NA | |
| Molecular Formula | C₁₄H₁₁N₅O₃ | |
| Molecular Weight | 297.09 (confirmed by MS) | |
| Ref. | NA | |
| Company, Cat. | NA | |
| Full name | N6-(3-oxo-1-propenyl)-2'-deoxyadenosine (synthetic adduct) | |
| Short name | M₁dA or OPdA | 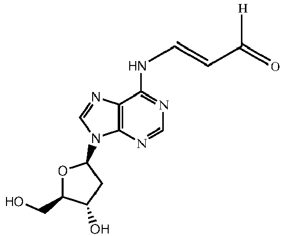 |
| CAS Number | NA | |
| Molecular Formula | C₁₃H₁₅N₅O₄ | |
| Molecular Weight | 305.11 (confirmed by MS) | |
| Ref. | Voulgaridou, Anestopoulos et al. 2011 | |
| | Marnett 2002 | |
| | Stone et al. 1990 | |
| Company, Cat. | NA | |
| Full name | 8-(9-((2*R*,4*S*,5*R*)-4-hydroxy-5-(hydroxymethyl) tetrahydrofuran-2-yl)-9*H*-purin-6-yl)-2-oxa-8-azabicyclo [3.3.1] nona-3,6-diene-4,6-dicarbaldehyde | |
| Short name | M3dA | 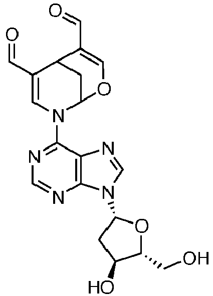 |
| CAS Number | NA | |
| Molecular Formula | C₁₉H₁₉N₅O₆ | |
| Molecular Weight | 413.13 | |
| Ref. | Stone et al. 1990 | |
| Company, Cat. | NA | |
| Full name | 1-(3-((2*R*,4*S*,5*R*)-4-hydroxy-5-(hydroxymethyl) tetrahydrofuran-2-yl)-3*H*-imidazo[2,1-*i*] purin-7-yl) heptan-2-one (synthetic adduct) | |
| Short name | Heptanone-1, *N*²-ε-dA or ONEdA | 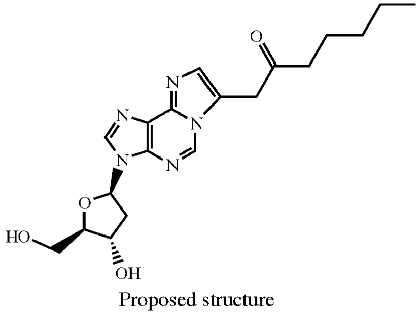 |
| CAS Number | NA | |
| Molecular Formula | C₁₉H₂₅N₅O₄ | |
| Molecular Weight | 389.21 | |
| Ref. | Geacintov and Broyde 2010 | |
| | Voulgaridou, Anestopoulos et al. 2011 | |
| | Kawai and Nuka 2018 | |
| Company, Cat. | NA | |
| Full name | 1-methylguanosine | 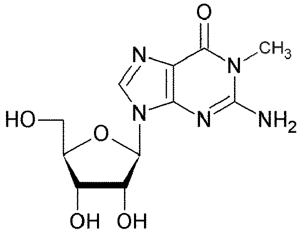 |
| Short name | m1G | |
| CAS Number | 2140-65-0 | |
| Molecular Formula | C₁₁H₁₅N₅O₅ | |
| Molecular Weight | 297.27 | |
| Ref. | Seidel, Brunner et al. 2006 | |
| Company, Cat. | Toronto Research Chemicals, B426593 | 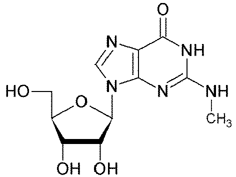 |
| Full name | N2-methylguanosine | |
| Short name | m2G | |
| CAS Number | 2140-77-4 | |
| Molecular Formula | C₁₁H₁₅N₅O₅ | |
| Molecular Weight | 297.27 | |
| Ref. | Seidel, Brunner et al. 2006 | |
| Company, Cat. | Santa Cruz, sc-215517 | |
| Full name | 7-methylguanosine | 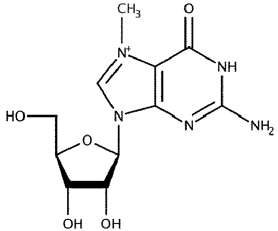 |
| Short name | m7G | |
| CAS Number | 20244-86-4 | |
| Molecular Formula | C₁₁H₁₇N₅O₅ | |
| Molecular Weight | 299.27 | |
| Ref. | NA | |
| Company, Cat. | Sigma-Aldrich, M0627 | |
| | | |
| Full name | 2'-O-methylguanosine | 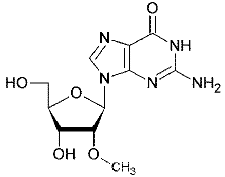 |
| Short name | Gm | |
| CAS Number | 2140-71-8 | |
| Molecular Formula | C₁₁H₁₅N₅O₅ | |
| Molecular Weight | 297.27 | |
| Ref. | NA | |
| Company, Cat. | Cayman, 21039 | |
| Full name | N2,N2-dimethylguanosine | 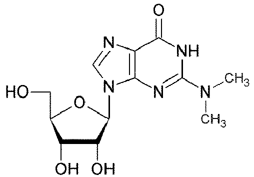 |
| Short name | m²₂G | |
| CAS Number | 2140-67-2 | |
| Molecular Formula | C₁₂H₁₇N₅O₅ | |
| Molecular Weight | 311.30 | |
| Ref. | Seidel, Brunner et al. 2006 | |
| Company, Cat. | NA | |
| Full name | O-beta-ribosyl(1"-2')-guanosine-5"-phosphate | 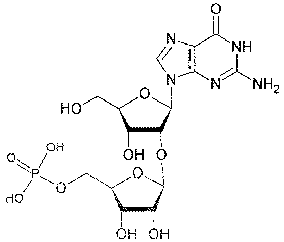 |
| Short name | Gr | |
| CAS Number | 131293-20-4 | |
| Molecular Formula | C₁₅H₂₂N₅O₁₂P₁ | |
| Molecular Weight | 495.34 | |
| Ref. | NA | |
| Company, Cat. | NA | |
| Full name | 3-((2*R*,3*R*,4*S*,5*R*)-3,4-di hydroxy-5-(hydroxymethyl )tetra hydrofuran-2-yl)-6,7-dihydroxy-7-methyl-6,7-dihydro-3*H*-imidazo[1,2-*a*]purin-9(5*H*)-one | |
| | one | 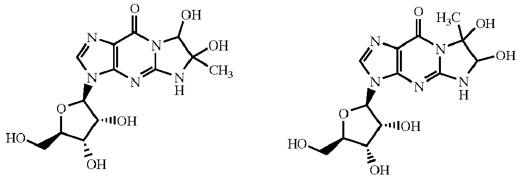 |
| Short name: | MGG | |
| CAS Number | NA | |
| Molecular Formula | C₁₃H₁₇N₅O₇ | |
| Molecular Weight | 355.11 | |
| Ref. | Richarme, Liu et al. 2017 | |
| | Kim, Park et al. 2017 | |
| Company, Cat. | NA | |
| Full name | 2-((6-oxo-6,7-dihydro-1 *H*-purin-2-yl)amino)propanoate | |
| Short name | CEdG or 2-(1-Carbohyethyl)guanine | 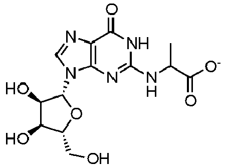 |
| CAS Number | NA | |
| Molecular Formula | C₁₃H₁₆N₅O₇ | |
| Molecular Weight | 354.11 | |
| Ref. | NA | |
| Company, Cat. | NA | |
| Full name | 3-((2*R*,5*R*)-4-hydroxy-5-(hydroxymethyl) tetrahydrofuran-2-yl)-7-(2-oxoheptyl)-3*H*-imidazo[1,2-a]purin-9(5*H*)-one (synthetic adduct) | |
| Short name | Heptanone-1,*N*²-ε-dG or ONEdG | 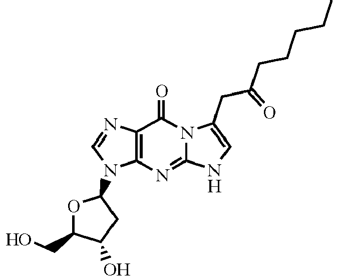 |
| CAS Number | NA | |
| Molecular Formula | C₁₉H₂₅N₅O₅ | |
| Molecular Weight | 403.19 | |
| Ref. | Geacintov and Broyde 2010 | |
| | Voulgaridou, Anestopoulos et al. 2011 | |
| | Kawai and Nuka 2018 | |
| Company, Cat. | NA | |
| Full name | 3-(2-deoxy-β-D-erythro-pentofuranosyl)pyrimido[1,2-α]purin-10(3H)-one | |
| | (synthetic adduct) | 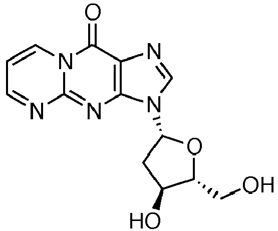 |
| Short name | M₁dG | |
| CAS Number | NA | |
| Molecular Formula | C₁₃H₁₃N₅O₄ | |
| Molecular Weight | 303.27 | |
| Ref. | Voulgaridou, Anestopoulos et al.2011 | |
| | Marnett 2002 | |
| | Wauchope, Beavers et al. 2015 | |
| | Riggins, Daniels et al. 2004 | |
| | Stone et al. 1990 | |
| Company, Cat. | NA | |
| Full name | N2-oxopropenyl-deoxyguanosine (synthetic adduct) | 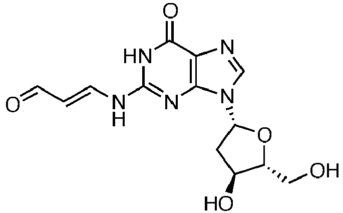 |
| Short name | N²OPdG | |
| CAS Number | NA | |
| Molecular Formula | C₁₃H₁₄N₅O₅ | |
| Molecular Weight | 303.28 | |
| Ref. | Voulgaridou, Anestopoulos et al. 2011 | |
| | Marnett 2002 | |
| | Wauchope, Beavers et al. 2015 | |
| | Riggins, Daniels et al. 2004 | |
| Company, Cat. | NA | |
| Full name | 3-((2*R*,4*S*,5*R*)-4-hydroxy-5-(hydroxymethyl)tetrahydrofuran-2-yl)-12-oxo-5,6,10,12-tetrahydro-3*H*-6,10-methano[1,3,5]oxadiazocino[5,4-*a*]purine-9-carbaldehyde | |
| Short name | M2dG | 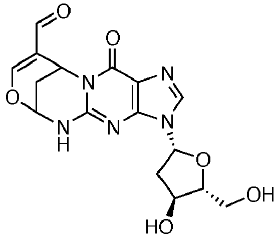 |
| CAS Number | NA | |
| Molecular Formula | C₁₆H₁₇N₅O₆ | |
| Molecular Weight | 375.12 | |
| Ref. | Stone et al. 1990 | |
| Company, Cat. | NA | |
| Full name | 2'-*O*-methylcytidine | 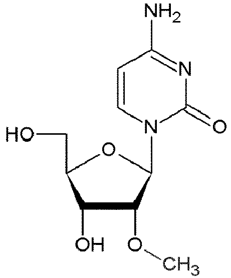 |
| Short name | Cm | |
| CAS Number | 2140-72-9 | |
| Molecular Formula | C₁₀H₁₅N₃O₅ | |
| Molecular Weight | 257.25 | |
| Ref. | NA | |
| Company, Cat. | TCI Chemicals, M2317 | |
| Full name | 3-methyluridine | 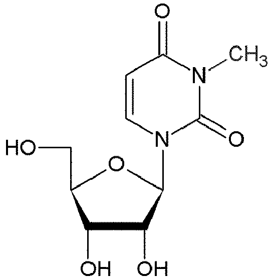 |
| Short name | m3U | |
| CAS Number | 2140-69-4 | |
| Molecular Formula | C₁₆H₁₄N₂O₆ | |
| Molecular Weight | 258.23 | |
| Ref. | NA | |
| Company, Cat. | NA | |
| | | |
| | | 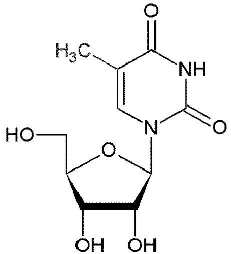 |
| Full name | 5-methyluridine | |
| Short name | m5U | |
| CAS number | 1463-10-1 | |
| Molecular Formula | C₁₆H₁₄N₂O₆ | |
| Molecular Weight | 258.23 | |
| Ref. | NA | |
| Company, Cat. | Sigma-Aldrich, 535893 | |
| Full name | 3,2'-O-dimethyluridine | 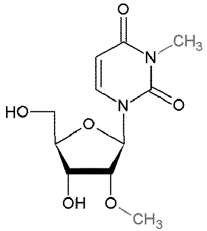 |
| Short name | m3Um | |
| CAS number | NA | |
| Molecular Formula | C₁₁H₁₆N₂O₆ | |
| Molecular Weight | 272.26 | |
| Ref. | NA | |
| Company, Cat. | NA | |
| Full name | Queuosine | 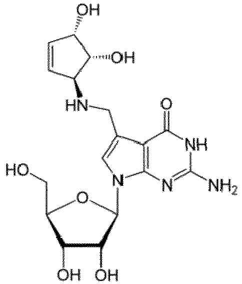 |
| Short name | Q | |
| CAS number | 57072-36-3 | |
| Molecular Formula | C₁₇H₂₃N₅O₇ | |
| Molecular Weight | 409.39 | |
| Ref. | NA | |
| Company, Cat. | Toronto Research Chemicals, N925205 | |
| Full name | Wybutosine | 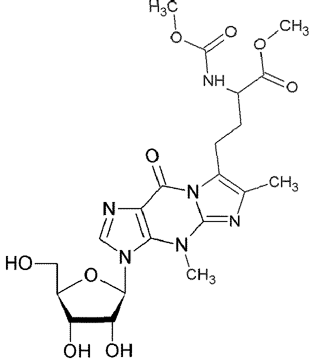 |
| Short name | yW | |
| CAS number | 55196-46-8 | |
| Molecular Formula | C₂₁H₂₈N₆O₉ | |
| Molecular Weight | 508.49 | |
| Ref. | NA | |
| Company, Cat. | NA | |
| | | 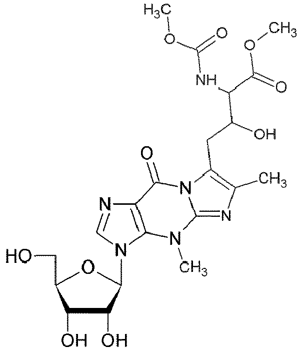 |
| Full name | Hydroxywybutosine | |
| Short name | OHyW | |
| CAS number | NA | |
| Molecular Formula | C₂₁H₂₈N₆O₁₀ | |
| Molecular Weight | 524.49 | |
| Ref. | NA | |
| Company, Cat. | NA | |
| Full name | 5-(3,4-dihydroxy-5-(hydroxymethyl)tetrahydrofuran-2-yl)pyrimidine-2,4(1*H*,3*H*)-dione | 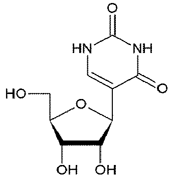 |
| Short name | Pseudouridine, Psi | |
| CAS number | 1445-07-4 | |
| Molecular Formula | C₉H₁₂N₂O₆ | |
| Molecular Weight | 244.20 | |
| Ref. | NA | |
| Company, Cat. | NA | |
| Full name | 6-((2*R*,4*S*,5*R*)-4-hydroxy-5-(hydroxymethyl)tetrahydrofuran-2-yl)-3-(2-oxoheptyl)-1,8a-dihydroimidazo[1,2-*c*]pyrimidin-5(6*H*)-one (synthetic adduct) | 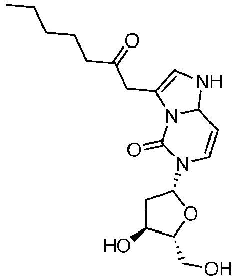 |
| Short name | Heptanone-1,*N*²-ε-dC or ONEdC | |
| CAS number | 1445-07-4 | |
| Molecular Formula | C₁₈H₂₅N₃O₅ | |
| Molecular Weight | 365.20 | |
| Ref. | Voulgaridou, Anestopoulos et al. 2011 Kawai and Nuka 2018 | |
| Company, Cat. | NA | |
| Full name | N4-(3-oxo-1-propenyl)-2'-deoxycytidine (synthetic adduct) | 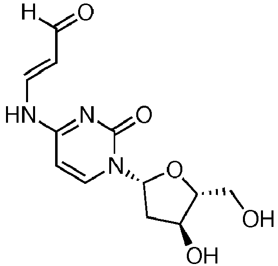 |
| Short name | M₁dC or OPdC | |
| CAS number | NA | |
| Molecular Formula | C₁₂H₁₅N₃O₅ | |
| Molecular Weight | 281.10 | |
| Ref. | Voulgaridou, Anestopoulos et al. 2011 | |
| | Marnett 2002 | |
| Company, Cat. | NA | |
| Full name | 8-(1-((2*R*,4*S*,5*R*)-4-hydroxy-5-(hydroxymethyl)tetrahydrofuran-2-yl)-2-oxo-1,2-dihydropyrimidin-4-yl)-2-oxa-8-azabicyclo[3.3.1]nona-3,6-diene-4,6 dicarbaldehyde | 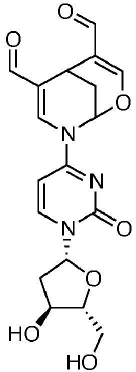 |
| Short name | M3dC | |
| CAS number | NA | |
| Molecular Formula | C₁₈H₁₉N₃O₇ | |
| Molecular Weight | 389.12 | |
| Ref. | NA | |
| Company, Cat. | NA | |
| Full name | 6-Methylmercaptopurine (2*R*,3*S*,4*R*,5*R*)-2-(hydroxymethyl)-5-(6-(methylthio)-9*H*-purin-9-yl)tetrahydrofuran-3,4-diol | |
| Short name | 6-MMP | |
| CAS number | 50-66-8 | |
| Molecular Formula | C₁₁H₁₄N₄O₄S | |
| Molecular Weight | 298.32 | |
| Ref. | NA | |
| Company, Cat. | Toronto Research Chemicals, M320270 | |
| Full name | 3-Methyladenine | 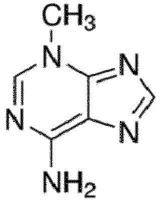 |
| Short name | 3MA | |
| CAS Number | 5142-23-4 | |
| Molecular Formula | C₆H₇N₅ | |
| Molecular Weight | 149.15 | |
| Company, Cat. | Cayman CAY-13242 | |
| Full name | N6-methyladenosine | 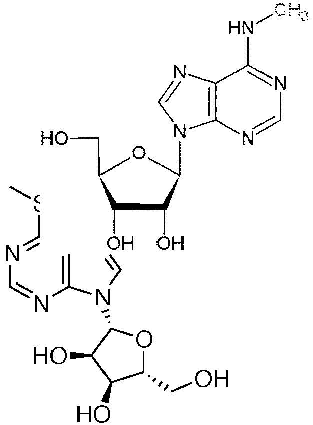 |
| Short name | m6A | |
| CAS Number | 1867-73-8 | |
| Molecular Formula | C₁₁O₄N₅H₁₅ | |
| Molecular Weight | 281 | |
| Company, Cat. | Toronto Research Chemicals, M275895 | |
| Full name | 6-Methylpurine | |
| Short name | MeP | 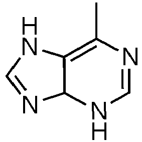 |
| CAS Number | 2004-03-7 | |
| Molecular Formula | C₆H₆N₄ | |
| Molecular Weight | 134.14 | |
| Company, Cat. | Sigma-Aldrich, M6502 | |
| | | |
| Full name | 6-(Dimethylamino)purine | 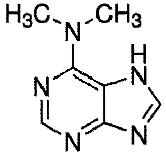 |
| Short name | 6DMAP | |
| CAS Number | 938-55-6 | |
| Molecular Formula | C₇H₉N₅ | |
| Molecular Weight | 163.18 | |
| Company | Sigma-Aldrich, D2629 | |
| | | |
| Full name | N6-(Δ2-Isopent-2-enyl) adenine | 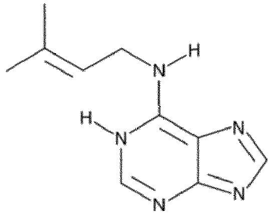 |
| Short name | i6Ade | |
| CAS Number | 2365-40-4 | |
| Molecular Formula | C₁₀H₁₃N₅ | |
| Molecular Weight | 203.2 | |
| Company, Cat. | Cayman, CAY-17906 | |
| Full name | 6-(γ,γ-Dimethylallylamino)purine | 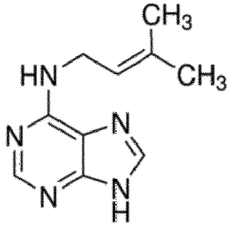 |
| Short name | 2iP | |
| CAS Number | 2365-40-4 | |
| Molecular Formula | C₁₀H₁₃N₅ | |
| Molecular Weight | 203.24 | |
| Company, Cat. | Sigma-Aldrich, D7660 | |
| | | |
| Full name | N1-Methyl-2'-deoxyguanosine | 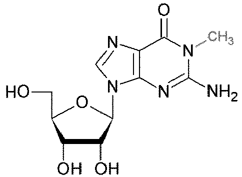 |
| Short name | N1MedG | |
| CAS Number | 5132-79-6 | |
| Molecular Formula | C₁₁H₁₅N₅O₄ | |
| Molecular Weight | 281.27 | |
| Company, Cat. | Toronto Research Chemicals, M293030 | |
| Full name | 1-Methylguanine | 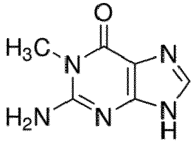 |
| Short name | MeG | |
| CAS Number | 938-85-2 | |
| Molecular Formula | C₆H₇N₅O | |
| Molecular Weight | 165.15 | |
| Company, Cat. | Sigma-Aldrich, 67070 | |
| Full name | N2-Methyl-2'-deoxyguanosine | 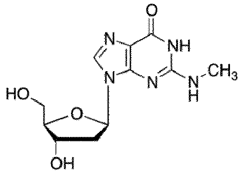 |
| Short name | N2MedG | |
| CAS Number | 19916-77-9 | |
| Molecular Formula | C₁₁H₁₅N₅O₄ | |
| Molecular Weight | 281.27 | |
| Company, Cat. | Toronto Research Chemicals, M293035 | |
| Full name | 7-Methyl-7-deaza-2'-deoxyguanosine | 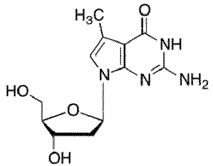 |
| Short name | 7Medeaza2dG | |
| CAS Number | 90358-21-7 | |
| Molecular Formula | C₁₂H₁₆N₄O₄ | |
| Molecular Weight | 280.28 | |
| Company, Cat. | Toronto Research Chemicals, M299590 | |
| Full name | O6-Methyl-2'-deoxyguanosine | 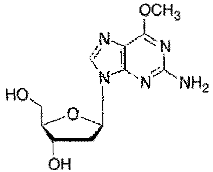 |
| Short name | O⁶MedG | |
| CAS Number | 964-21-6 | |
| Molecular Formula | C₁₁H₁₅N₅O₄ | |
| Molecular Weight | 281.27 | |
| Company, Cat. | Toronto Research Chemicals, M293040 | |
| Full name | N2-Ethyl-2'-deoxyguanosine | 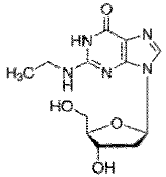 |
| Short name | N2EtdG | |
| CAS Number | 101803-03-6 | |
| Molecular Formula | C₁₂H₁₇N₅O₄ | |
| Molecular Weight | 295.29 | |
| Company, Cat. | Sigma-Aldrich, N3289 | |
| Full name | 5'-Deoxy-5'-(methylthio)adenosine | 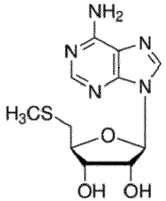 |
| Short name | MTA | |
| CAS Number | 2457-80-9 | |
| Molecular Formula | C₁₁H₁₅N₅O₃S | |
| Molecular Weight | 297.33 | |
| Company, Cat. | Sigma-Aldrich, D5011 | |
| Full name | N6-Methyl-2'-deoxyadenosine | 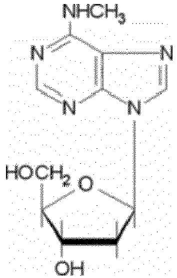 |
| Short name | N⁶MedAdo | |
| CAS Number | 2002-35-9 | |
| Molecular Formula | C₁₁H₁₅N₅O₃ | |
| Molecular Weight | 265.27 | |
| Company, Cat. | Sigma-Aldrich, M2389 | |
| Full name | N6-(2-Hydroxyethyl)-2'-deoxyadenosine | |
| Short name | N6HEdG | 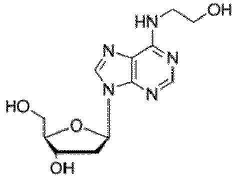 |
| CAS Number | 137058-94-7 | |
| Molecular Formula | C₁₂H₁₇N₅O₄ | |
| Molecular Weight | 295.29 | |
| Company, Cat. | Toronto Research Chemicals, H941985 | |
| Full name | O6-(2-Hydroxyethyl)-2'-deoxyguanosine | 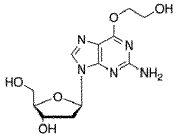 |
| Short name | O6HEdG | |
| CAS Number | 111447-35-9 | |
| Molecular Formula | C₁₂H₁₇N₅O₅ | |
| Molecular Weight | 311.29 | |
| Company, Cat. | Toronto Research Chemicals, H942020 | 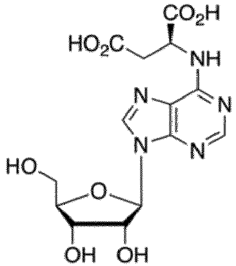 |
| Full name | N6-Succinyl Adenosine | |
| Short name | SAdo | |
| CAS Number | 4542-23-8 | |
| Molecular Formula | C₁₄H₁₇N₅O₈ | |
| Molecular Weight | 383.31 | |
| Company, Cat. | Toronto Research Chemicals, S688825 | |
| Full name | 2-(2-((3-((2*R*,4*S*,5*R*)-4-hydroxy-5-(hydroxymethyl)tetrahydrofuran-2-yl)-3,7-dihydropyrimido[2,1-*i*]purin-7-yl)oxy)cyclopropylidene)malonaldehyde | 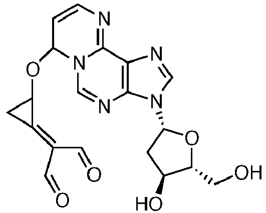 |
| Short name | M3dA-2 | |
| CAS Number | NA | |
| Molecular Formula | C₁₉H₁₉N₅O₆ | |
| Molecular Weight | 413.13 | |
| Company, Cat. | NA | |

### References

Geacintov, N. E. and S. Broyde (2010). The chemical biology of DNA damage. Weinheim, Wiley-VCH.
Ishiwata, S., K. Itoh, T. Yamaguchi, N. Ishida and M. Mizugaki (1995). "Comparison of serum and urinary levels of modified nucleoside, 1-methyladenosine, in cancer patients using a monoclonal antibody-based inhibition ELISA." Tohoku J Exp Med 176(1): 61-68.
Kawai, Y. and E. Nuka (2018). "Abundance of DNA adducts of 4-oxo-2-alkenals, lipid peroxidation-derived highly reactive genotoxins." J Clin Biochem Nutr 62(1): 3-10.
Kim, C. S., S. Park and J. Kim (2017). "The role of glycation in the pathogenesis of aging and its prevention through herbal products and physical exercise." J Exerc Nutrition Biochem 21(3): 55-61.
Marnett, L. J. (2002). "Oxy radicals, lipid peroxidation and DNA damage." Toxicology 181-182: 219-222.
Richarme, G., C. Liu, M. Mihoub, J. Abdallah, T. Leger, N. Joly, J. C. Liebart, U. V. Jurkunas, M. Nadal, P. Bouloc, J. Dairou and A. Lamouri (2017). "Guanine glycation repair by DJ-1/Park7 and its bacterial homologs." Science 357(6347): 208-211.
Riggins, J. N., J. S. Daniels, C. A. Rouzer and L. J. Marnett (2004). "Kinetic and thermodynamic analysis of the hydrolytic ring-opening of the malondialdehyde-deoxyguanosine adduct, 3-(2'-deoxy-beta-D-erythro-pentofuranosyl)-pyrimido[1,2-alpha]purin-10(3H)-one." J Am Chem Soc 126(26): 8237-8243.
Seidel, A., S. Brunner, P. Seidel, G. I. Fritz and O. Herbarth (2006). "Modified nucleosides: an accurate tumor marker for clinical diagnosis of cancer, early detection and therapy control." Br J Cancer 94(11): 1726-1733.
Stone, K., M.B. Ksebati, and L.J. Marnett (1990). "Investigation of the Adducts Formed by Reaction of Malondialdehyde with Adenosine" Chem. Res. Toxicol. 3: 33-38.
Voulgaridou, G. P., I. Anestopoulos, R. Franco, M. I. Panayiotidis and A. Pappa (2011). "DNA damage induced by endogenous aldehydes: current state of knowledge." Mutat Res 711(1-2): 13-27.
Wauchope, O. R., W. N. Beavers, J. J. Galligan, M. M. Mitchener, P. J. Kingsley and L. J. Marnett (2015). "Nuclear Oxidation of a Major Peroxidation DNA Adduct, M1dG, in the Genome." Chem Res Toxicol 28(12): 2334-2342.

All exemplary compounds were shown to interact with MR1, elicited MR1-restricted T cell responses and stabilized MR1 expression on cells as evidenced by results obtained by the assay methods as shown in the examples.

### Examples

### Example 1: Methods

### Human blood samples

Blood specimens for T cell cloning, FACS analysis and antigen-presentation assays were obtained from the University Hospital Basel after informed consent, according to protocols EKNZ 2017-01888, which received ethical approval from the Swiss authorities (EKNZ, Ethics Committee North-West & Central Switzerland).

### Cell lines

The cell lines used as antigen-presenting cells (APCs) in this study are A375 (ATCC CRL-1619), THP-1 (ATCC TIB-202), A375-MR1 and THP1-MR1, previously generated and described (Lepore et al. 2017 ibid.). Cells were free from Mycoplasma as evaluated by PCR analysis on DNA samples.

### Isolation and culturing of human T cells

All previously established human T cell clones were maintained in culture as described (Lepore, M., et al. (2014). Nat Commun 5: 3866). Newly described MR1T cells were isolated from the peripheral blood of healthy individuals. Briefly, after PBMC separation by density gradient centrifugation (Lymphoprep, StemCell Technologies, Cat#07851), T cells were purified by negative selection using EasySep Human T Cell Enrichment Kit (StemCell Technologies, Cat#19051) and stimulated with irradiated (80 Gray) APCs (ratio 2:1) and antigen once a week for three weeks. Human rIL-2 (5 U/mL, Peprotech, Cat#200-02) was added at day +2 and +5 after each stimulation. Twelve days after the last stimulation, cells were washed and co-cultured overnight with APCs (ratio 2:1) in the presence or absence of compounds.

CD3⁺CD69⁺CD137^{high} cells were then FACS sorted and stable long-term lines established. Clones were obtained by limiting dilution in the presence of phyto-haemagglutinin (1 µg/mL, Remel, Cat#30852801 HA16), human rIL-2 (100 U/mL, Peprotech) and irradiated PBMC (5×10⁵ cells/mL). In some experiments, MR1T cell lines and clones were generated from sorted CD3⁺, compound-loaded MR1-tetramer⁺ cells, upon stimulation with APCs (ratio 2:1). T cell clones were periodically re-stimulated following the previously described protocol (Lepore et al. 2014).

### TCR gene transfer

The TCRα and β functional cDNA from MR1T clones were cloned into a modified version of the Lenti expression vector (Addgene, Cat#52962). Endogenous TCR-deficient SKW-3 or J76 cells were transduced with virus particle-containing supernatants generated as previously described (Lepore et al. 2017). Transduced cells were selected by FACS sorting based on CD3 expression, when necessary.

### Preparation and purification of compounds

Compounds M₃ADE, M1dC, MGdA, MGG, m6,6A, io6A, m6t6A, Ar(p), M1dA, M3dA, ONEdA, m2,2G, Gr, CEdG, ONEdG, M1dG, N²OPdG, M2dG, m3U, m3Um, yW, OHyW, Psi, ONEdC, M3dC were synthesized in house and subsequently purified before use with cells. All other compounds were purchased from different vendors as indicated in **Table 1.**

M₃ADE was produced by mixing 2'-deoxyadenosine monohydrates (25mM, Sigma, Cat#D7400) with malondialdehyde tetrabutylammonium salt (100mM, Sigma, Cat#63287) in acetic acid, pH 4.0 (2.2% v/v in water, Sigma, 695092). The mixture was incubated for 4 days at 37°C under 400 rpm shaking. M₃ADE crude compound preparation was subjected to Solid Phase Extraction (Phenomenex, Strata® C18-E, Cat#8B-S001-MFF). M₃ADE was eluted with 30% acetonitrile. Further HPLC purification was performed by reversed-phase HPLC with a C18 Pyramid column (Macherey-Nagel, Cat# 762204.40) as follows. Mobile phase A: deionized water; mobile phase B: 95% methanol in deionized water. Flow rate 1.25 mL/min. Elution gradient: time 0 min, A 100%; time 1 min, A 98%; time 43 min A 50%; time 46 min A 50%; time 47 min A 100%; time 56 min A 100%. Biologically active HPLC-separated compounds were collected for mass spectrometric and NMR analyses.

M1dC was produced by mixing 2'-deoxycytidine (25mM, Sigma, Cat#D3897) with malondialdehyde tetrabutylammonium salt (100mM). The mixture was incubated for 18 h at 70°C under 400 rpm shaking. M1dC crude compound preparation was subjected to Solid Phase Extraction as above. M1dC was eluted with 20% acetonitrile.

Further HPLC purification was performed by reversed-phase HPLC with a C18 Pyramid column (Macherey-Nagel, Cat# 762204.40) as follows. Mobile phase A: deionized water; mobile phase B: 95% methanol in deionized water. Flow rate 1.25 mL/min. Elution gradient: time 0 min, A 100%; time 1 min, A 98%; time 43 min A 50%; time 46 min A 50%; time 47 min A 100%; time 56 min A 100%. Biologically active HPLC-separated compounds were collected for mass spectrometric and NMR analyses.

MGdA was produced by mixing 2'-deoxyadenosine monohydrates (25mM, Sigma, Cat#D7400) with methylglyoxal solution (100mM, Sigma, Cat#M0252) in acetic acid, pH 4.0 (2.2%, v/v in water). The mixture was incubated for 5 days at 70°C under 400 rpm shaking. MGdA crude compound preparation was processed by using three Solid Phase Extraction cartridges. MGdA was first purified on C18 cartridge (Phenomenex, Strata® C18-E, Cat#8B-S001-MFF) and eluted with 100% methanol. C18-eluted MGdA was dried under nitrogen stream then loaded on QMA cartridge (Accell Plus QMA, Cat#WAT054640) and eluted with formic acid, pH 2.0 (2% v/v in water, AppliChem, Cat#A3858). The pH of QMA-eluted MGdA was adjusted to 7.0 with NaOH, and purified with 50% methanol on Phenyl cartridge (Phenomenex, Strata® Phenyl, Cat#8B-S006-KDG). Further HPLC purification was performed by reversed-phase HPLC with a C18 Pyramid column (Macherey-Nagel, Cat# 762204.40) as follows. Mobile phase A: deionized water; mobile phase B: Acetonitrile. Flow rate 1.25 mL/min. Elution gradient: time 0 min, A 100%; time 1 min, A 75%; time 8.5 min A 65%; time 9.5 min A 0%; time 11.5 min A 0%; time 11.6 min A 100%, time 14.6 min A 100%. Biologically active HPLC-separated compounds were collected for mass spectrometric and NMR analyses.

MGG was produced by mixing guanosine (100mM, Sigma, Cat#G6752) with methylglyoxal solution (100mM) in DMSO (33.3%, v/v in water, Sigma Cat#D4540). The mixture was incubated for 2 h at 70°C under 400 rpm shaking. Further HPLC purification was performed by reversed-phase HPLC with a C18 Pyramid column (Macherey-Nagel, Cat# 762272.100) as follows. Mobile phase A: deionized water; mobile phase B: 95% methanol in deionized water. Flow rate 5 mL/min. Elution gradient: time 0 min, A 97%; time 2.5 min, A 97%; time 30 min A 87%; time 32.5 min A 0%; time 37.5 min A 0%; time 40 min A 100%, time 52.5 min A 100%. Biologically active HPLC-separated compounds were collected for mass spectrometric and NMR analyses.

### Evaluation of the biological activity of compounds

Cell surface MR1 modulation was measured on THP-1 MR1 cells (10⁵ cells/well) after incubation for 3 h at 37°C in the presence or absence of compounds (3 doses each). Ac-6-FP (100 µM, Schircks Laboratories Cat#11.418) was used as positive control compound for MR1 surface upregulation. MR1 expression was evaluated by staining with mouse mAbs antihuman-MR1 APC-labeled (IgG2a,k clone 26.5, Biolegend Cat#361108) and subtracting the background staining with APC-labeled mouse IgG2a,k isotype control antibodies (Biolegend Cat#400220), which was always below 300 MFI.

Competition assays were performed by incubating APCs (10⁵ cells/well) with compounds (3 doses each) for 2 h at 37°C, then adding the antigen for each clone of interest at optimal concentration (≥ EC₅₀) and incubating for 2 additional h before the addition of T cells (10⁴ cells/well). As positive control for competition with antigens, Ac-6-FP was used (100 µM). Supernatants were collected after 24 h for cytokine analysis measured by ELISA.

T cell activation assays were performed using MR1T cells (5×10⁴/well) co-cultured with indicated APCs (10⁵/well) for 24 h in 125 µL volume in triplicates. In some experiments, anti-MR1 mAbs (purified and endotoxin-free mouse IgG2a, clone 26.5 (Lepore et al. 2014)) or mouse IgG2a isotype control mAbs (LEAF, Biolegend, Cat#401504) (both at 30 µg/mL) were added and incubated for 30 min at 37°C prior to the addition of T cells. In some experiments, APCs were pre-incubated with 6-FP or Ac-6-FP (Schircks Laboratories, Cat#11.415 or 11.418, respectively) or with 6,7-dimethyl-8-D-ribityllumazine (RL-6,7-diMe, 150 µM, OTAVA Chemicals) for 4 h before co-culture with T cells.

In some experiments, APCs were fixed by mixing one volume of glutaraldehyde (1% in water, Sigma, Cat#G5882) with one volume of APCs (less than 3×10⁶ cells/mL). Fixed APCs were incubated with antigens for 4 h at 37°C prior to the addition of T cells. MR1T cells (10⁵/well unless otherwise indicated) were co-cultured with indicated APCs cells (2×10⁵/well unless otherwise indicated) for 18 h in 150 µL volume in triplicates.

Activation experiments with plate-bound recombinant human β2m-MR1 were performed by coating β2m-MR1 onto 96-well plates (4 µg/mL) and loading with compounds for 4 h at 37°C before washing twice and adding T cells.

Supernatants were collected after 24 h for cytokine analysis.

### Cytokine Analysis

The following human cytokines were assessed by ELISA using specific mAbs: GM-CSF (purified clone BVD2-23B6 and biotinylated clone BVD2-21C11, Biolegend Cat#502202 and 502304, respectively), IFN-γ (purified clone MD-1 and biotinylated clone 4S.B3, Biolegend Cat#507502 and 502504, respectively), IL-13 (purified clone JES10-5A2 and biotinylated clone SB126d, SouthernBiotech Cat#10125-01 and 15930-08, respectively).

### MR1 protein production and tetramerization

Soluble recombinant MR1 monomers were generated as previously described (Kjer-Nielsen, L., et al. (2012). Nature 491(7426): 717-723). Briefly, nucleotide sequences coding for the soluble portions of the mature human MR1 (GenBank accession number NM_001531) and mature human β2m (GenBank accession number NM_004048.3) were cloned into the bacterial expression vector pET23d (Novagen, Cat#69748-3). Transformed *E*. *coli* BL21(DE3)pLysS were then grown to OD₆₀₀ₙₘ 0.4-0.6 before induction with 0.6M isopropyl β-D-1-thiogalactopyranoside (Sigma-Aldrich, Cat#10724815001). After 4 h of further culture, cells were lysed and inclusion bodies were cleaned, purified and fully denatured in 8M Urea, 10mM EDTA, 0.1mM DTT for subsequent storage at -80°C.

For protein refolding, MR1 heavy chain (4mM), β2m (2mM) and compound (15mM) were added to 1L refolding buffer containing 0.4M L-arginine, 100mM Tris pH 8.0, 2mM EDTA that was cooled to 4°C and supplemented with 5mM reduced glutathione and 0.5mM oxidized glutathione immediately beforehand. After 3 days, the refold mix was concentrated down to 1mL and the refolded compound-bearing MR1 was purified by HPLC using Superdex 75 10/300 GL (GE Healthcare, Cat#17517401) and MonoQ 5/50 (GE Healthcare, Cat#17516601) columns.

Correct protein conformation was confirmed by performing a plate-bound activation of the MR1T cell clone DGB129. Refolded MR1-compound protein was serially diluted in PBS (1.5 - 100 µg/mL) and added to a high-protein-binding plate (Nunc, Cat#439454) for 2 h at 37°C. The wells were washed thoroughly with PBS before addition of 5×10⁴ cells that were left overnight at 37°C. IL-13 ELISA was then used as an activation readout. The functional monomer was then biotinylated using BirA-500 biotinylation kit (Avidity, Cat#Bulk BirA) overnight at 4°C. Excess biotin was removed using S75 10/30 (GE Healthcare, 29148721) gel filtration before tetramerization with phycoerythrin (PE)-streptavidin (Prozyme, Cat#PJRS25) in a 4:1 molar ratio.

As control MR1 tetramers, human MR1-5-OP-RU and human MR1-6-FP labelled with APC, PE or AlexaFluor488 were used (The MR1 tetramer technology was developed jointly by Dr. James McCluskey, Dr. Jamie Rossjohn, and Dr. David Fairlie, and the material was produced by the NIH Tetramer Core Facility as permitted to be distributed by the University of Melbourne).

### Immunofluorescence Staining

Cell surface labeling was performed using standard protocols. All mAbs for staining were used at 5 µg/mL. Biotinylated mAbs were revealed with streptavidin-PE (Biolegend, Cat#405204), - Alexa Fluor 488 (Biolegend, Cat#405235), or -Brilliant violet 421 (Biolegend, Cat#405226) all at 2 µg/mL.

When staining with MR1-tetramers, the cells were pre-treated with Dasatinib (50nM, Sigma-Aldrich, Cat#CDS023389) for 30 min at 37°C before first adding anti-CD8 mAbs (Biolegend, clone RP8-TA BV711) for 20 min at room temperature (RT), then 50 µg/mL MR1-tetramer for a further 20 min at RT without washing. All remaining mAbs were then added for a further 20 min at RT without washing. The cells were then washed in PBS before acquisition at the flow cytometer.

Samples were acquired on LSR Fortessa flow cytometer with the FACS Diva software (Becton Dickinson). Cell sorting experiments were performed using an Influx instrument (Becton Dickinson). Dead cells and doublets were excluded on the basis of forward scatter area and width, side scatter, and DAPI staining (Sigma, Cat#D9542). All data were analyzed using FlowJo (LLC).

### Data Analysis

Data analysis, statistical tests and visualization were conducted using GraphPad Prism.

Cytokine secretion assays and flowcytometry data were analyzed using Unpaired Student's t-test.

A p value ≤0.05 was considered statistically significant (*p ≤0.05, **p ≤0.01, ***p ≤0.005).

### Results

### Compounds stimulating MR1T cells

The inventors' previous results showed that MR1T cells recognize MR1 molecules complexed with ligands present in tumor cells (Lepore et al. 2017, ibid.). The purification of cellular extracts of THP-1 cells, led them to the identification of compounds which can be defined as modified nucleobase and nucleobase adducts.

The inventors screened commercially available compounds using three types of biological assays. All three assays are based on the capacity of the compounds to bind MR1 and i) to modulate surface expression levels of MR1, ii) to activate in a specific manner at least one MR1T cell clone, or iii) to compete with the stimulatory compounds, thus affecting the response of MR1T cell clones. The biologically active compounds are reported in **Table 1.**

Examples of compound reactivity for each of the three functional assays indicated above, are illustrated in **Figures 1-3****.**

### Detection and sorting of MR1T cells by MR1 tetramer staining

As proof of concept to demonstrate the use of these novel compounds for the detection and capture of MR1T cells, the inventors selected the compound M₃ADE. MR1 molecules containing the M₃ADE were generated by *in vitro* refolding of human recombinant soluble MR1 produced in bacteria. Properly refolded MR1 monomers loaded with M₃ADE were purified by gel filtration chromatography (**Figure 4a**), and their capacity to stimulate MR1T cells, in the absence of APCs, was tested using plate-bound assays (**Figure 4b**). The correctly refolded MR1-M₃ADE complexes were then biotinylated and tetramerized using streptavidin. MR1-M₃ADE tetramer specificity was validated through the *ex vivo* capture of reactive T cells from PBMCs, followed by their expansion and generation of T-cell clones which maintained the original characteristics of tetramer reactivity and the expected compound specificity. As example, one such clone, named AVA34, was confirmed by positive staining with MR1-M₃ADE tetramers but negative with MR1-5-OP-RU tetramers (**Figure 5a**). Abrogation of MR1-M₃ADE tetramer binding by pre-incubation with anti-Vβ antibodies (anti-Vβ8 mAbs), further confirmed TCR specificity of the tetramer staining (**Figure 5a**). The clone AVA34 was also specifically activated upon re-exposure to M₃ADE but no other compounds presented by MR1⁺APCs **(****Figure 5b****).** After these validation experiments, MR1-M₃ADE tetramers were used to detect reacting MR1T cells from freshly isolated PBMC of healthy donors (**Figure 6**). MR1-M₃ADE tetramer positive cells were readily detected and their frequency ranged from -0.005% to 0.097% (mean 0.027%) of CD3⁺ cells, similar to conventional HLA-restricted T cells and to the frequencies previously reported for MR1T cells using different strategies (Lepore et al. 2017). To promote the accumulation of carbonylated nucleosides, in some experiments, THP-1 cells were treated with drugs that increase the amounts of cellular carbonyls. These drugs (daidzin, disulfiram, oleanoic acid, ellagic acid) induced a strong T cell response in MR1T cell clones (Figure 7, panels a, b, c, d, e). In further experiments THP-1 cells were treated with the adenosine deaminase inhibitor EHNA to induce accumulation of adenosine-containing adducts or with mycophenolic acid, which inhibits the enzyme inosine-5'-monophosphate dehydrogenase (IMPDH), thus inducing an increase of inosine and adenosine-containing nucleosides adducts. Both these drugs induced a strong stimulation of MR1T cells (Figure 7, panel f and g).

## Claims

1. A method for modulating an interaction between an MR1 polypeptide and an MR1-specific T cell receptor molecule, said method comprising contacting said MR1 polypeptide with
a. a compound described by any one of the following general formulas: 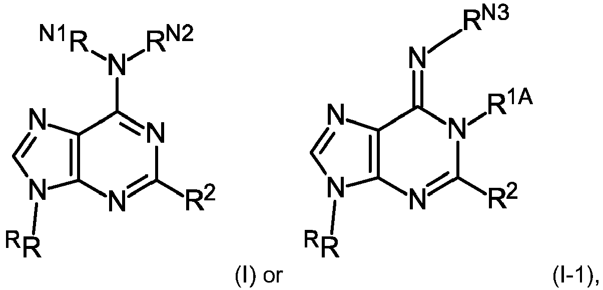 or 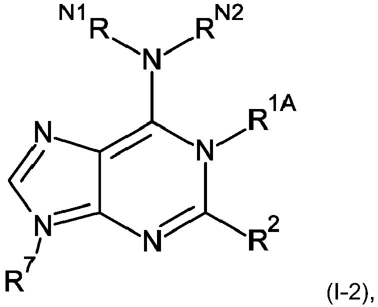 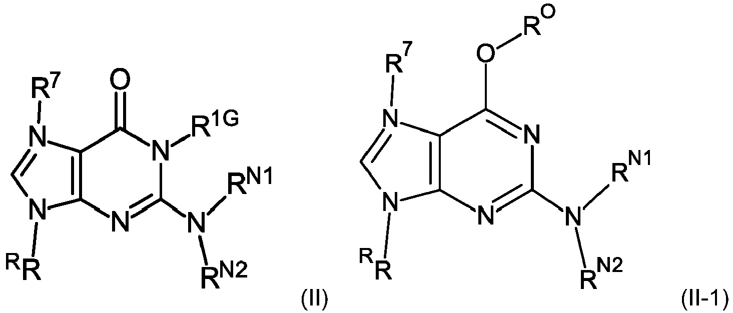 or 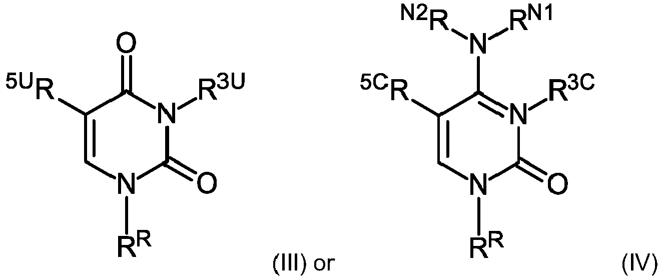 or or 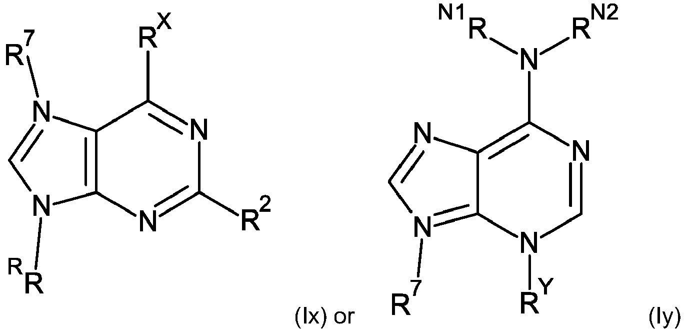 wherein
- R^{1A} is H or methyl, R^{1G} is H or methyl;
- R^{N3} is H or methyl;
- R² is selected from H, methyl and -S-methyl;
- R^{3U} and R^{5U} are selected from H and methyl; R^{3C} may be methyl or no atom attached to the N, and R^{5C} is selected from H and methyl;
- R^{N1} and R^{N2} are both H or C₁ to C₃ alkyl, or R^{N1} is H or C₁ to C₃ alkyl (particularly R^{N1} is H or methyl) and R^{N2} is selected from a C₁-C₆ alkyl and a C₂-C₆ alkylene, wherein the alkyl or alkylene is unsubstituted or substituted with carbonyl, carboxyl and/or hydroxyl, particularly R^{N2} is selected from a methyl, 2-hydroxyethyl, 1,2-dicarboxy-ethyl, threonylcarbamoyl, isopent-2-enyl, cis-hydroxyisopent-2-enyl, 3-oxo-1-propenyl, and hexa-1,3,5-triene-1,1,3-tricarbaldehyde;
- or R^{N1}, R^{N2} and the nitrogen together form a 2-oxa-8-azabicyclo [3.3.1] nona-3,6-diene-4,6-dicarbaldehyde bi-annular system;
or
- R^{N3} and R^{1A} together, or R^{N2} and R^{3C} together, or R^{N1} and R^{1G} together form an unsubstituted or C₄ to C₁₆-2-oxo-alkyl-substituted or ω-carboxy-2-oxo-alkylsubstituted imidazole ring, or
R^{N3} and R^{1A} together, or R^{N2} and R^{3C} together, or R^{N1} and R^{1G} together are - C(CH₃)OH-CHOH- or C(R')OH- CH₂-CHOH- with R' being selected from H, CH₃, CH(OH)C₂H₅, C₂H₅ and C₄H₉;
or R^{N1} and R^{1G} form a pyrimidine, or R^{N1} and R^{1G} or R^{N3} and R^{1A} form a 12-oxo-5,6,10,12-tetrahydro-3H-6,10-methano[1,3,5]oxadiazocine ring system, or R^{N1} and R^{1G} form a 2-oxa-6,8-diazabicyclo [3.3.1] nona-3-ene-4-carbaldehyde bi-annular system;
and
- R⁷ is selected from an unbound electron pair and methyl, particularly R⁷ is an unbound electron pair;
- R^{O} is selected from H, unsubstituted or hydroxyl-substituted C₁-C₅ alkyl or C₂-C₅ alkylene,
- R^{X} is selected from SH, C₁-C₅ alkyl, C₂-C₅ alkylene, and C₁-C₅ S-alkyl;
- R^{Y} is selected from C₁-C₅ alkyl, C₂-C₅ alkylene, and C₁-C₅ O-alkyl;
- R^{R} is selected from H, 1'-ribosyl, 2'-deoxy-1'-ribosyl, 5'-phospho-1'-ribosyl, 5'-methylthio-1'-ribosyl, 1'-(2'-O-ribosyl-5"-phosphate)ribosyl, 1'-(2'-O-methyl)ribosyl;
with the proviso that adenine, adenosine, deoxyadenosine, guanine, guanosine, deoxyguanosine, uracil, uridine, deoxyuridine, thymine, thymidine, deoxythymidine, cytosine and cytidine and deoxycytidine are not encompassed by the general formulas I, II, III and IV,
b. or with a compound selected from queuosine, wybutosine, hydroxywybutosine or pseudouridine, or with (2R,3S,4R,5R)-2-(hydroxymethyl)-5-(6-(methylthio)-9H-purin-9-yl)tetrahydrofuran-3,4-diol.

2. The method according to claim 1, wherein R^{R} is described by the general formula (V) or (V-S) 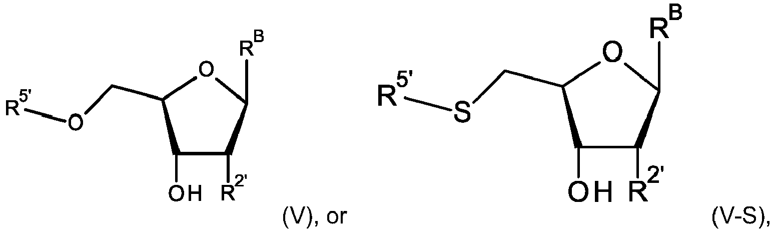 wherein R^{B} is the bond connecting the moiety to the N⁹ nitrogen of I, Ia or II, or to the N¹ nitrogen of II or IV, R^{2'} is selected from H, OH, OCH₃, O-ribosyl and O-ribosyl-5"-phosphate, and R^{5'} is selected from H and PO₃²⁻;
particularly wherein R^{R} is described by the general formula (V),
more particularly wherein R^{R} is described by the general formula (Va) 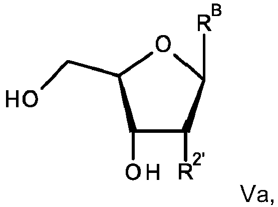
even more particularly wherein R^{R} is described by the general formula (Vb) or (Vc) or (Vd):

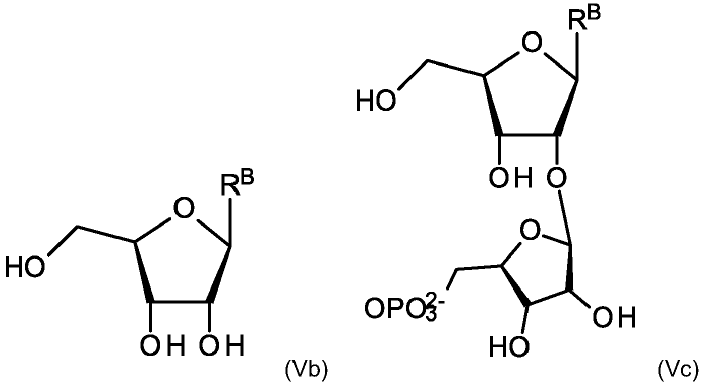

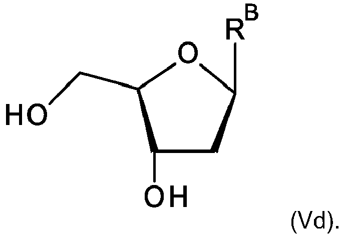

3. The method according to claim 1 or 2, wherein the compound is described by any one of the following general formulas 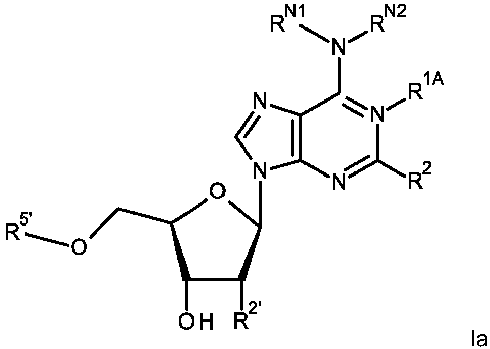 or 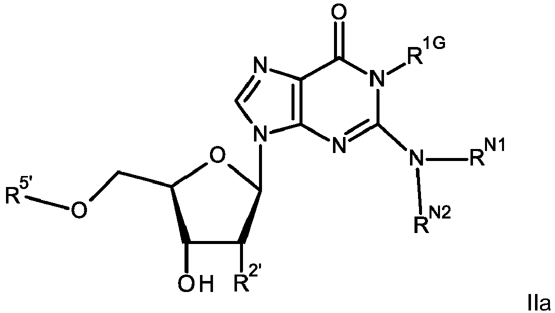
- wherein R^{N1}, R^{N2}, R^{1A}, R^{1G}, R^{2'} and R^{5'} can have the meaning indicated above,
- and wherein R^{R} has the meaning as indicated in claim 1 or 2.

4. The method according to any one of claims 1 to 3, wherein the compound is described by
a. formula (I) wherein R² is S-methyl and R^{N1} and R^{N2} are both H;
b. formula (I) wherein R² is methyl and R^{N1} and R^{N2} are both H; or
c. formula (Ia) wherein R^{1A} is methyl, R² is H and R^{N3} is H; or
d. formula (Ia) wherein R^{1A} is methyl, R² is methyl and R^{N3} is H; or
e. formula (Ia) wherein R^{1A} is methyl, R² is S-methyl and R^{N3} is H; or
f. formula (I) wherein R² is H, one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl; or
g. formula (I) wherein R² is S-methyl and one of R^{N1} and R^{N2} is selected from H and methyl, and the other one of R^{N1} and R^{N2} is selected from methyl, ethan-2-ol, 1,2-dicarboxy-ethyl, 3-methylbut(2)enyl, 3-hydroxymethylbut(2)enyl and threonylcarbamoyl;
h. formula (I) wherein R² is methyl and one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl;
i. formula (II) wherein R^{1G} is methyl, R⁷ is an unbound electron pair and R^{N1} and R^{N2} are both H; or
j. formula (II) wherein R^{1G} is methyl, R⁷ is methyl and R^{N1} and R^{N2} are both H; or
k. formula (II) wherein R^{1G} is H, R⁷ is methyl and R^{N1} and R^{N2} are both H; or
l. formula (II) wherein R^{1G} is methyl, R⁷ is an unbound electron pair and one of R^{N1} and R^{N2} is selected from H and methyl, and the other one of R^{N1} and R^{N2} is selected from methyl, ethyl, 3-methylbut(2)enyl, 3-hydroxymethylbut(2)enyl and threonylcarbamoyl; or
m. formula (II) wherein R^{1G} is methyl, R⁷ is methyl and one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl; or
n. formula (II) wherein R^{1G} is H, R⁷ is methyl and one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl; or
o. formulas (I-IM or II-IM), with R^{IM} selected from H, CH₂COCₙH₍₂ₙ₊₁₎ with n from 3 to 7 (part. n=5), and CH₂CO(CH₂)ₘCOO⁻ with m from 3 to 9 (part. 7) 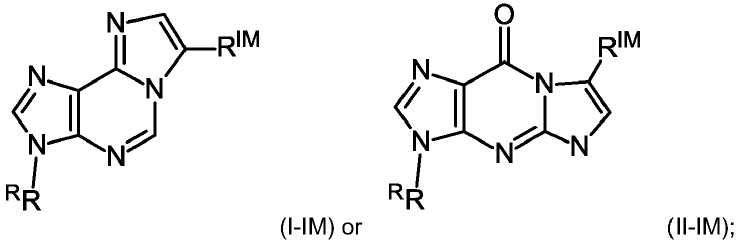 or
p. formulas (II-c), (II-d), (II-e) (II-f) 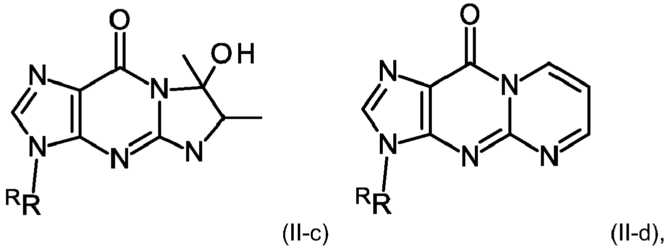 or 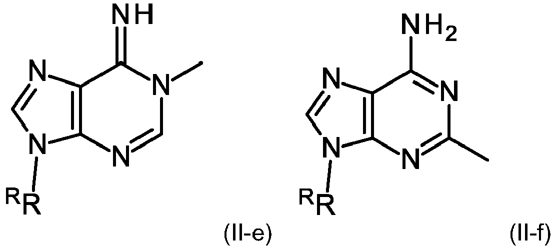
q. formulas (II-g) or (II-h), wherein R² is selected from H, methyl and S-methyl: 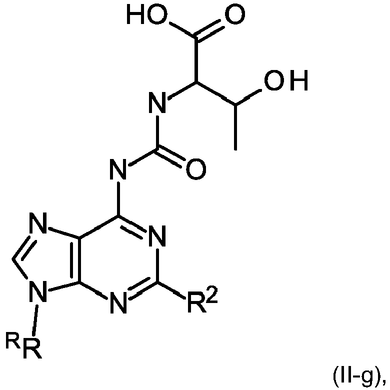 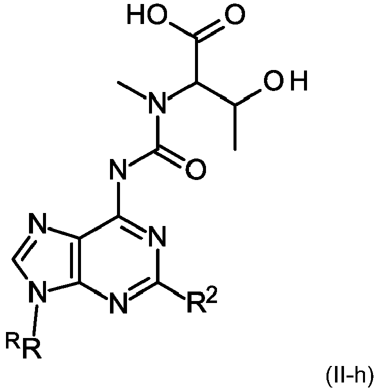
r. formulas (II-i) (II-j), wherein R^{N1} is selected from H and methyl and wherein R² is selected from H, methyl and S-methyl: 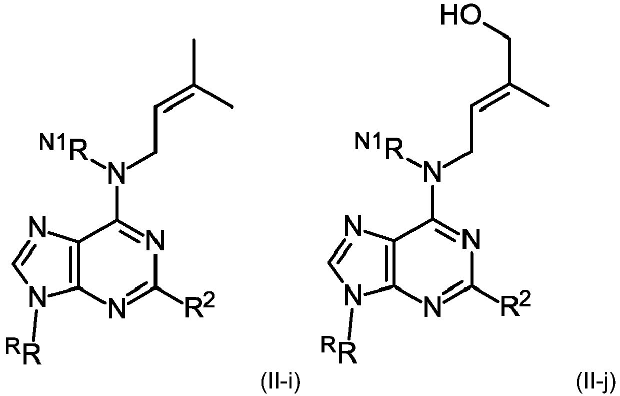
s. formula (III), wherein R^{3U} is H and R^{5U} is methyl,
t. formula (III), wherein R^{3U} is methyl and R^{5U} is H,
u. formula (III), wherein R^{3U} and R^{5U} are both methyl,
v. formula (IV), wherein R^{3C} and R^{5C} are H, one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl;
w. formula (IV), wherein R^{3C} is methyl and R^{5C} is H, one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl; or
x. formula (IV), wherein R^{3C} is H and R^{5C} is methyl, one of R^{N1} and R^{N2} is selected from H and methyl, and the other one of R^{N1} and R^{N2} is selected from methyl, 3-methylbut(2)enyl, 3-hydroxymethylbut(2)enyl and threonylcarbamoyl;
y. formula (Ix), wherein R² is H, R⁷ is an unbound electron pair, and R^{X} is selected from methyl and S-methyl;
z. formula (Iy), wherein R⁷ is an unbound electron pair, R^{N1} and R^{N2} are both H, and R^{Y} is selected from C₁-C₅ alkyl, particularly R^{Y} is methyl;
aa. formula (I), wherein R² is H, R^{N1} is H, R^{N2} is 3-oxo-1-propenyl;
bb. formula (I-a),
(I-a)
cc. formula (II-k),
(II-k)
dd. formula (I-2), wherein R^{1A}, R^{N1} and R² are H, and R⁷ is an unbound electron pair, and R^{N2} is isopent-2-enyl, or cis-hydroxyisopent-2-enyl,
ee. formula (I), wherein R^{N1} and R² are both H, and R^{N2} is isopent-2-enyl, or cis-hydroxyisopent-2-enyl,
ff. formula (II-1), wherein R^{O} is methyl or ethan-2-ol, R⁷ is an unbound electron pair, and R^{N1} and R² are both H;
gg. formula (I-b),
(I-b)
and wherein in each formula, R^{R} has the meaning as indicated in claim 1 or 2.

5. The method according to any one of claims 1 to 4, wherein the compound is selected from
a. 1-methyladenosine
b. 2-methyladenosine
c. 2'-O-methyladenosine
d. N6,N6-dimethyladenosine
e. N6-threonylcarbamoyladenosine
f. N6-isopent-2-enyladenosine
g. N6-(cis-hydroxyisopent-2-enyl) adenosine
h. 2-methylthio-N6-(cis-hydroxyisopent-2-enyl) adenosine
i. 2-methylthio-N6-isopent-2-enyladenosine
j. N6-methyl-N6-threonylcarbamoyladenosine
k. 2'-O-ribosyladenosinephosphate
l. (3*Z*,5*E*)-6-((9*H*-purin-6-yl) amino) hexa-1,3,5-triene-1,1,3-tricarbaldehyde
m. N6-(3-oxo-1-propenyl)-2'-deoxyadenosine
n. 8-(9-((2*R*,4*S*,5*R*)-4-hydroxy-5-(hydroxymethyl) tetrahydrofuran-2-yl)-9*H-*purin-6-yl)-2-oxa-8-azabicyclo [3.3.1] nona-3,6-diene-4,6-dicarbaldehyde
o. 1-(3-((2*R*,4*S*,5*R*)-4-hydroxy-5-(hydroxymethyl) tetrahydrofuran-2-yl)-3*H-*imidazo[2,1-*i*] purin-7-yl) heptan-2-one
p. 1-methylguanosine
q. N2-methylguanosine
r. 7-methylguanosine
s. 2'-O-methylguanosine
t. N2,N2-dimethylguanosine
u. 2'-O-ribosylguanosine
v. 3-((2*R*,3*R*,4*S*,5*R*)-3,4-dihydroxy-5-(hydroxymethyl)tetrahydrofuran-2-yl)-6,7-dihydroxy-7-methyl-6,7-dihydro-3*H*-imidazo[1,2-*a*]purin-9(5*H*)-one
w. 2-((6-oxo-6,7-dihydro-1*H*-purin-2-yl)amino)propanoate
x. 3-((2*R*,5*R*)-4-hydroxy-5-(hydroxymethyl) tetrahydrofuran-2-yl)-7-(2-oxoheptyl)-3*H*-imidazo[1,2-a]purin-9(5*H*)-one
y. 3-(2-deoxy-β-D-erythro-pentofuranosyl)pyrimido[1,2-α]purin-10(3H)-one
z. N2-oxopropenyl-deoxyguanosine
aa. 3-((2*R*,4*S*,5*R*)-4-hydroxy-5-(hydroxymethyl)tetrahydrofuran-2-yl)-12-oxo-5,6,10,12-tetrahydro-3*H*-6,10-methano[1,3,5]oxadiazocino[5,4-*a*]purine-9-carbaldehyde
bb. 2'-*O*-methylcytidine
cc. 3-methyluridine
dd. 5-methyluridine
ee. 3,2'-*O*-dimethyluridine
ff. 6-((2*R*,4*S*,5*R*)-4-hydroxy-5-(hydroxymethyl)tetrahydrofuran-2-yl)-3-(2-oxoheptyl)-1,8a-dihydroimidazo[1,2-*c*]pyrimidin-5(6*H*)-one
gg. N4-(3-oxo-1-propenyl)-2'-deoxycytidine
hh. 8-(1-((2*R*,4*S*,5*R*)-4-hydroxy-5-(hydroxymethyl)tetrahydrofuran-2-yl)-2-oxo-1,2-dihydropyrimidin-4-yl)-2-oxa-8-azabicyclo[3.3.1]nona-3,6-diene-4,6 dicarbaldehyde
ii. (2*R*,3*S*,4*R*,5*R*)-2-(hydroxymethyl)-5-(6-(methylthio)-9*H*-purin-9-yl)tetrahydrofuran-3,4-diol
jj. 6-methylmercaptopurine
kk. 3-methyladenine
ll. N6-methyladenosine
mm. 6-methylpurine
nn. 6-(dimethylamino)purine
oo. N6-(Δ2-isopentenyl) adenine
pp. 6-(γ,γ-dimethylallylamino)purine
qq. N1-methyl-2'-deoxyguanosine
rr. 1-methylguanine
ss. N2-methyl-2'-deoxyguanosine
tt. 7-methyl-7-deaza-2'-deoxyguanosine
uu. O6-methyl-2'-deoxyguanosine
vv. N2-ethyl-2'-deoxyguanosine
ww. 5'-deoxy-5'-(methylthio)adenosine
xx. N6-methyl-2'-deoxyadenosine
yy. N6-(2-hydroxyethyl)-2'-deoxyadenosine
zz. O6-(2-hydroxyethyl)-2'-deoxyguanosine
aaa. N6-Succinyl adenosine
bbb. 2-(2-((3-((2*R*,4*S*,5*R*)-4-hydroxy-5-(hydroxymethyl)tetrahydrofuran-2-yl)-3,7-dihydropyrimido[2,1-*i*]purin-7-yl)oxy)cyclopropylidene)malonaldehyde.

6. The method according to any one of the preceding claims, wherein the method is selected from
a. a method for the identification and isolation of T cells reactive to the MR1 ligand compound, and
b. a method of diagnosis, wherein a sample obtained from a patient is analyzed with regard to the presence of an MR1 ligand compound as identified herein.

7. A compound as specified in any one of claims 1 to 5 for use in prophylaxis or treatment of a disease associated with an aberrant or absent MR1-specific T cell response, particularly in treatment of cancer **characterized by** tumor cells expressing MR1.

8. The compound for use according to claim 7, wherein the compound is co-administered with an anticancer drug, and/or checkpoint modulator agent.

9. The compound for use according to claim 4 to 5, wherein the compound is co-administered with a preparation comprising (transgenic) MR1-reactive T cells and/or a polynucleotide expression vector encoding MR1.

10. A method for identification of a T cell reactive to a MR1 ligand compound as specified in any one of claims 1 to 5, said method comprising the steps
providing a preparation of T cells reactive to /capable of specifically recognizing MR1;
a. contacting said preparation of T cells with a complex comprising isolated MR1 associated to said compound;
b. isolating a T cell that is specifically reactive to said MR1 ligand compound in an isolation step.

11. A compound selected from disulfiram, daidzin, ellagic acid, oleanoic acid, erythro-9-(2-hydroxy-3-nonyl) adenine (EHNA), and mycophenolic acid for prophylaxis or therapy of a disease associated with aberrant or lacking MR1 expression, particularly treatment or prevention of recurrence of cancer disease associated with tumor cells expressing MR1.
